# EUROPEAN PATENT SPECIFICATION

(11) **EP 0 392 791 B1**
(45) Date of publication and mention of the grant of the patent: **02.08.1995**
(21) Application number: 90303818.0
(22) Date of filing: 10.04.1990
(51) Int. Cl.: C07H 19/10, C07H 19/20, A61K 31/70, C07F 9/6558, C07F 9/6561

(54) **Antiviral compounds**
Antivirale Verbindungen
Composés antiviraux

(30) Priority: 13.04.1989 GB 8908355
(43) Date of publication of application: 17.10.1990
(73) Proprietor: BRITISH TECHNOLOGY GROUP LIMITED, London SE1 6BU (GB)
(72) Inventor: Walker, Richard Thomas, Selly Oak, Birmingham B29 4BJ (GB); Jones, Albert Stanley, Yardley, Birmingham B46 1NL (GB)
(74) Representative: Scott, Susan Margaret

(56) References cited:
- EP-A- 0 284 405
- DE-A- 2 009 834
- CHEMICAL AND PHARMACEUTICAL BULLETIN, vol. 28, no. 10, pages 2915-2923; M. Saneyoshi et al.: "Synthetic nucleosides and nucleotides. XVI. Synthesis and biological evaluations of a series of 1-beta-D-arabino-furanosylcytosine 5,-alkyl or arylphosphates"
- CHEMISCHE BERICHTE., vol.108, no. 9,1975, pages 2857-2871; A.MYLES et al.: "Nucleotide, 1. Synthese und Eigenschaften von Thymidylyl-(3, to 3,)-,-(3, to 5,)- und -(5, to 5,) - thymidin"

## Description

This invention relates to antiviral compounds, the use thereof, processes for the production of such compounds and intermediates useful in such processes.

Whilst the antiviral compound azidothymidine (AZT) is used clinically to combat the Human Immunodeficiency Virus (HIV) it suffers from drawbacks, for example, toxicity to bone marrow cells. Alternatives to AZT which have been proposed include the dinucleoside phosphates of EP-A-0284405 of structure 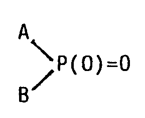
where A and B are nucleoside residues attached through the 5' position. The synthesis of dinucleoside phosphates containing a thymidine residue attached through the 5' position is also described by Myles et al., Chemische Berichte, 1975, 108, 2857-2871.

A different group of compounds has now been found which offers the promise of reduction in such toxicity shown by AZT.

Accordingly, the present invention comprises a compound of formula (I): 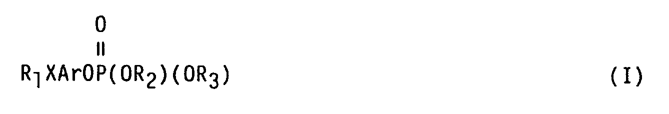
in which formula R₁ represents an alkyl group, which is preferably a C₁-C₆ alkyl group,
Ar represents a substituted or unsubstituted phenylene group,
X represents -SO₂- or -CO- and R₂ and R₃, which though usually identical may be different, represent moieties of formula (a), (b), (c), (d), (e), (f), (g), (h) or (i): 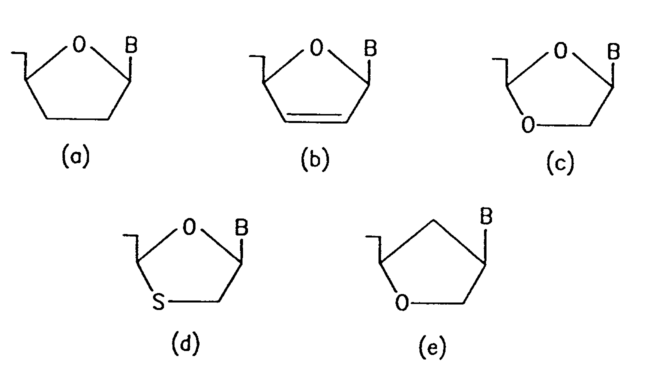 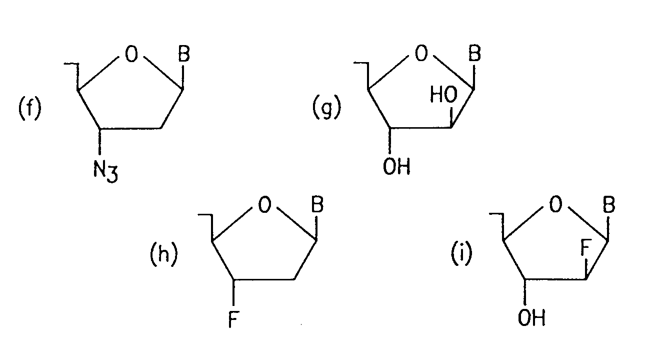
wherein B represents the residue of a nucleoside base of formula (A), (G), (C), (H) or (T): 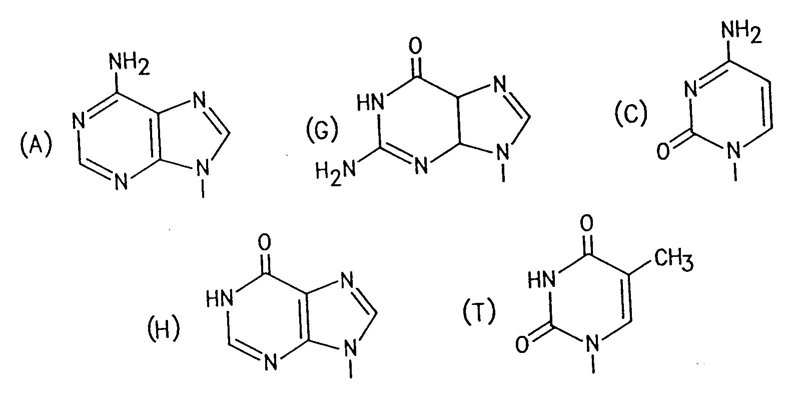
provided that when R₂ and R₃ both represent the moiety of formula (a) then B represents the residue of a nucleoside base which is of formula (A), (G), (C) or (H), the compound optionally being in the form of a pharmaceutically acceptable salt, e.g. a hydrochloride. It will be appreciated that (A), (G), (C), (H) and (T) represent the residues respectively of adenine, guanine, cytosine, hypoxanthine and thymine.

Typically, Ar represents a phenylene group in which the relative disposition of the group R₁X and phosphate substituents is mutually para, the ring usually carrying no further substituents.

When, however, Ar represents a substituted phenylene group, the substituents are selected with due attention to any level of toxicity shown by the corresponding phenol, R₁XArOH, produced on hydrolysis of the compound of formula (I). Thus, when Ar does not represent the preferred unsubstituted phenylene group, the substituents on a substituted phenylene group, which may be up to four in number, are preferably selected from halogen e.g. chlorine, fluoroalkyl e.g. trifluoromethyl, alkoxy e.g. C₁-C₄ alkoxy, fluoroalkoxy, carboalkoxy e.g. C₁-C₆ carboalkoxy, amino and amido. The alkyl group R₁ is generally unbranched and is typically a methyl group whilst X preferably represents a sulphonyl group.

Moieties R₂ and R₃ of especial interest include: (i) -(x) and particularly (i), (v), (vi) and (ix). 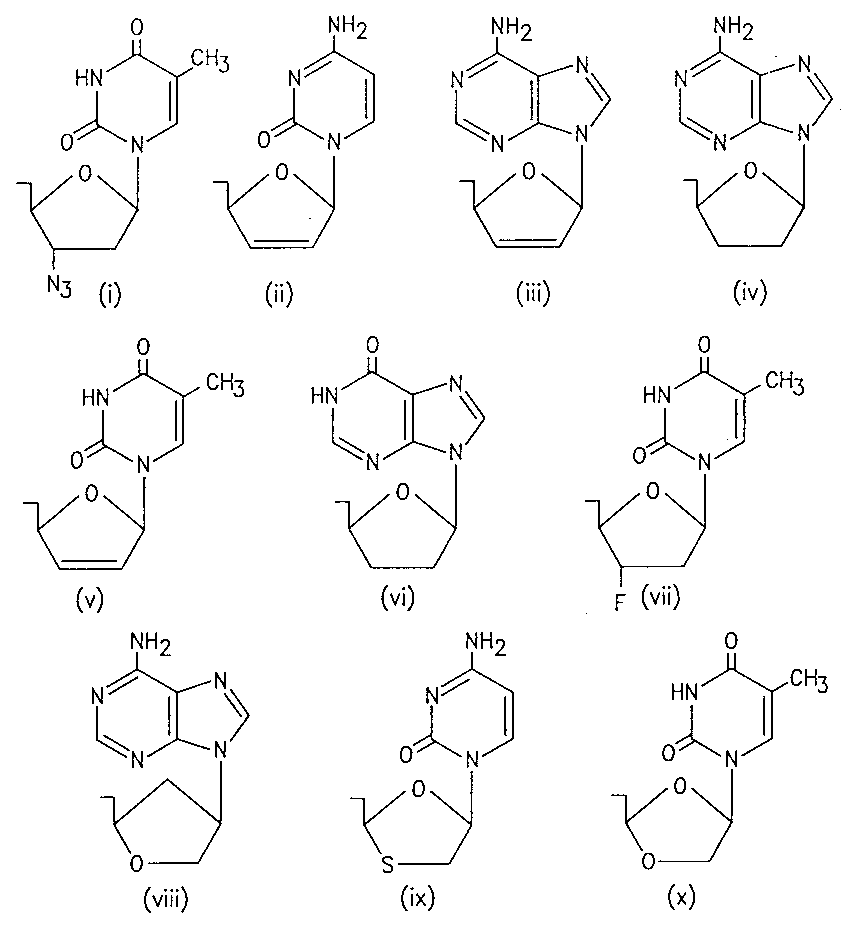
It will be appreciated that the moieties (i) -(vi) are found in compounds which may be represented by abbreviated nomenclature as (i) AZT, (ii) d⁴C, (iii) d⁴A, (iv) d²A, (v) d⁴T, (vi) ddI.

Compounds of the present invention in which R₂ and R₃ are identical may be produced in accordance with a further aspect thereof by reaction between a phosphorodihalidate of formula (III), R₁XArOP(O)Y₂, in which R₁, X and Ar correspond to those groups in the compound of formula (I) and Y represents halogen (e.g. chlorine) and a compound of formula R₂OH in which R₂ corresponds to the identical groups R₂ and R₃ in the compound of formula (I), (e.g. azidothymidine) or a derivative of the compound of formula R₂OH, e.g. a derivative in which a group in the nucleoside base is protected, as may be the free amino group in cytosine by acetylation. The reaction is usually conducted in the presence of a base e.g. 1-methylimidazole and is typically conducted in an aprotic solvent such as acetonitrile.

Alternatively, when X represents SO₂, compounds of formula I may be produced in accordance with a further aspect of the present invention by oxidation of a compound of formula IV: R₁SArOP(O)(OR₂)(OR₃) or of formula (V): R₁SOArOP(O)(OR₂)(OR₃), oxidation typically being carried out with per acid such as 3-chloroperbenzoic acid.

The present invention further includes within its scope intermediate compounds of formula (IV) and formula (V).

Compounds of the present invention find application in the treatment or prophylaxis of human retrovirus infections and particularly Human Immunodeficiency Virus (HIV) infection which gives rise to Acquired Immune Deficiency Syndrome (AIDS).

Accordingly, in a further aspect the invention comprises a compound of formula I for use in therapy and in a yet further aspect of the present invention the use of a compound of formula (I) in the manufacture of a medicament for use in the treatment or prophylaxis of a human retrovirus infection, particularly HIV, such as Acquired Immuno Deficiency Syndrome.

The dosage form and amount can be readily established by reference to known treatment or prophylactic regimens. In general however the dosage of the compound of formula (I) will be lower than the corresponding amount of AZT and usually lies within the range about 50 to about 800 mg.

While it is possible for the active compound of formula (I) or pharmaceutically acceptable salt thereof to be administered alone, it is preferable to present the active compound in a pharmaceutical formulation. Formulations of the present invention, for medical use, comprise the active compound together with one or more pharmaceutically acceptable carriers thereof and, optionally, any other ingredients which may be therapeutic per se, synergistic with the compound of formula I, or both. Carrier(s) must be pharmaceutically acceptable in the sense of being compatible with the other ingredients of the formulation and not deleterious to the recipient thereof.

The present invention therefore further provides a pharmaceutical composition comprising a compound of formula (I) (in the form of the free base or a pharmaceutically acceptable acid addition salt) together with a pharmaceutically acceptable carrier thereof.

The formulations include those suitable for oral, rectal, topical or parenteral (including subcutaneous, intramuscular and intravenous) administration.

The formulations may conveniently be presented in unit dosage form and may be prepared by any of the methods well known in the art of pharmacy. All methods include generally the step of bringing the active compound into association with a carrier which constitutes one or more accessory ingredients. Usually, the formulations are prepared by uniformly and intimately bringing the active compound into association with a liquid carrier or with a finely divided solid carrier or with both and then, if necessary, shaping the product into desired formulations.

Formulations of the compounds of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught. The active compound may also be presented as a bolus, electuary or paste.

A tablet may be made by compression or moulding, optionally with one or more accessory ingredients. Compressed tablets may be prepared by compressing, in a suitable machine, the active compound in a free-flowing form such as a powder or granulas, optionally mixed with a binder, lubricant, inert diluent, surface active or dispersing agent. Moulded tablets may be made by moulding, in a suitable machine, a mixture of the powdered active compound with any suitable carrier.

A syrup may be made by adding the active compound to a concentrated, aqueous solution of a sugar, for example sucrose, to which may be added any accessory ingredient. Such accessory ingredient(s) may include flavourings, an agent to retard crystallisation of the sugar or an agent to increase the solubility of any other ingredient, such as a polyhydric alcohol for example glycerol or sorbitol.

Formulations for rectal administration may be presented as a suppository with a usual carrier such as cocoa butter.

Formulations suitable for parental administration conveniently comprise a serile aqueous preparation of the active compound which is preferably isotonic with the blood of the recipient.

In addition to the aforementioned ingredients, formulations of this invention, for example ointments, creams and the like, may include one or more accessory ingredient(s) selected from diluents, buffers, flavouring agents, binders, surface active agents, thickeners, lubricants, preservatives (including antioxidants) and the like.

The present invention is illustrated by the following Example:

### Example 1

### Preparation of 4-(methylsulphonyl)phenyl bis (3'-azido thymidin-5'-yl) phosphate

### A. 4-(Methylsulphonyl)phenyl phosphorodichloridate.

4-(Methylthio)phenyl phosphorodichloridate. To a solution of freshly distilled phosphoryl chloride (45 ml, 0.5 mol) and 1-methylimidazole (0.15 ml), is added 4-(methylthio)phenol (14, 0.1 mol) and the solution is heated under reflux for 20 h. The excess of phosphoryl chloride is removed by distillation and the residue distilled under reduced pressure to give the product (11 g, 42% yield); bp 135-142°C (2 mm Hg) ¹H NMR (CDCl₃) δ 2.39 (3H, s, SCH₃), 7.22 (4 H, s, phenyl).

4-(Methylsulphonyl)phenol. To a solution of 4-(methylthio)phenol (7.0 g, 0.05 mol) in 30% aqueous methanol (100 ml) at 0°C is added a solution of sodium periodate (10.7 g, 0.05 mmol) and the resulting suspension is stirred for 30 min. Water (500 ml) is then added and the precipitate removed by filtration. The filtrate is cooled to 4°C and a further portion of sodium periodate (10.7 g, 0.05 mmol) added and the resulting suspension stirred for 48 h when a further portion of sodium periodate (5.35 g, 0.025 mmol) is added. After stirring for a further 18 h, the precipitate is removed by filtration, the filtrate extracted with ether which is evaporated to dryness and the residue is purified on a silica column using chloroform methanol, 9:1, as eluent to give the title compound (2.75 g, 32% yield).

4-(Methylsulphonyl)phenyl phosphorodichloridate. 4-(Methylsulphonyl)phenol (3.0 g, 17 mmol) is heated under reflux with freshly distilled phosphoryl chloride (13.35 ml, 87 mmol) and 1-methylimidazole (0.05 ml) for 20 h. The excess of phosphoryl chloride is removed by distillation and the residue is distilled under reduced pressure to give the title compound (bp 185°C, 1 mm Hg) as a yellow oil which solidifies on cooling (500 mg, 10% yield).

### B. 4-(methylsulphonyl)phenyl bis(3'-azidothymidin-5'-yl)phosphate.

4(methylsulphonyl)phenyl phosphorodichloridate (52mg 0.18 mmol), 1-methylimidazole (0.08ml 0.92mmol) and dry acetonitrile (3ml.) are stirred for 5 minutes at room temperature under dry nitrogen. The addition of azido thymidine (3'-azidothymidine; 3'-azido-3'-deoxythymidine; AZT) (80mg 0.3mmol) in 1ml of dry acetonitrile follows. The resulting suspension is then stirred overnight at room temperature. Thin Layer Chromatography (Tlc) of the reaction mixture shows only ca. 25% of the slower moving spot in CHCl₃:MeoH (9:1). At this stage another equivalent of 4-(methylsulphonyl)phenyl phosphorodichloridate and 1-methylimidazole in dry acetonitrile is added and the reaction mixture is stirred for a further 48 hours. Tlc then shows ca. 90% conversion to the slower moving component. After addition of phosphate buffer (15ml. pH6.0) the mixture is extracted with chloroform (4 x 10ml). The chloroform extracts are washed with water and then dried over magnesium sulphate. The chloroform is evaporated under reduced pressure and the residue is applied, pre-absorbed onto silica gel. to a short silica gel column (80g, type 7734). The column is eluted with chloroform : methanol (9:1). The appropriate fractions are concentrated to give a white solid (115mg., 52%).
NMR Spectrum:
(¹H)δ(d₆DMSO): 11.36(2H,S,NH), 7.95(2H,d,phenyl) 7.5S(2H,d,H-6), 7.45(2H,d,phenyl), 6.14(2H,t,-H-1¹), 4.4S(2H,m,H-3¹), 4.04(2H,m,H-4¹), 3.42(4H,m,H-S¹), 3.21(3H,S,SO₂CH₃) 2.44(4H,m,H-2¹), 1.71(6H,S, CH₃)
Elemental Analysis:
Found: C, 42.9; H, 4.5; N, 18.9; C₂₇H₃₁N₁₀O₁₂P5 requires C, 43.2; H, 4.16; N, 18.66%.
Mass Spectrum:
M/Z 751 (M+H)⁺. 773 (M+Na)⁺.

### Example 2

### Preparation of 4-(Methylsulphonyl)phenyl bis(3-azido thymidin-5'-yl)phosphate via 4-(methylthio)phenyl analogue.

4-Methylthio)phenyl bis(3'-azidothymidin-5'-yl)phosphate (144 mg, 0.2mmol, prepared by reaction of 4-(methylthio)phenyl phosphorodichloridate (Example 1A) with azido thymidine) is dissolved in dry ethanol and cooled to 0°C. A solution of 3-chloroperoxybenzoic acid (107mg, 0.6 mmol) in dry ethanol (15ml) is added dropwise with stirring over 15 minutes. The resulting solution is stored overnight at 5°C. After this period, Tlc (chloroform-methanol (9.1)) shows ca. 90% conversion to a slower-moving component. The solvent is evaporated under reduced pressure and the residue is applied, pre-absorbed onto silica gel, to a silica gel column (10g, type 9385). The column is eluted with chloroform-methanol (9:1). The appropriate fractions are concentrated and purified further using the chromatotron (2mm plate, same solvent system). The product is isolated as a white solid (105mg. 70%).

A sample of the compound 4-(methylsulphonyl)phenyl bis 3'-azidothymidin-5'-yl phosphate is shown by HPLC analysis in reverse phase chromatography to consist of 4 components : azidothymidine (AZT) as a minor component, 4-(methylsulphonyl) phenyl bis 3' azidothymidin-5'-yl phosphate, the latter compound without the side chain on the ester linkage and an unidentified component.

The sample, considered of adequate quality for antiviral testing is assayed as follows:

### Anti-HIV Testing

The assays are carried out in 96 well (microtitre) panels using the MT4 cell line, infected with IOTCID50 of HIV 3B. The antiviral activity and cytotoxicity of each compound is assayed simultaneously. Three compounds are screened on each panel. Each compound is tested at 100.0, 10.0, 1.0 and 0.1»M, unless otherwise stated. AZT is included in each assay as a positive control at 10.0, 1.0, 0.1 and 0.01»M.

The antiviral activity (in infected cells) and cytotoxicity (to uninfected cells) of each compound is determined by measuring the number of viable cells remaining after 5 days incubation of 37°C and comparing them with infected or uninfected controls. The number of viable cells is determined by the addition of the tetrazolium dye MTT. MTT uptake and conversion to a blue Formazan derivative has been shown to be linear with viable cell number. Following MTT addition, the cells are solubilised with acidified isopropanol and the extent of MTT conversion is measured spectrophotometrically.

Antiviral activity is apparent through the ability of compounds to protect the cells from virus induced cytopathic effect. The result is reported as the percentage of cells protected at a given drug concentration.

The results of the assay are shown in the Table

**TABLE**

| | % Protection | | | | | | |
|---|---|---|---|---|---|---|---|
| Concentration of BTG 1704: | 100 | 10 | 1.0 | 0.1 | 0.01 | 0.001»M | MTC |
| Antiviral Activity: | 13% | 79% | 91% | 91% | 21% | 9% | 100»M |

It can be seen from the Table that concentrations of BTG 1704 i.e. the compound of Example 2 between 0.1 and 10»M offers significant protection of MT4 cells from HIV-1 cytopathic effect. The toxic concentration of the drug is estimated to be about 100»M. This is not however a quantitative test for cytotoxicity.

### Example 3

### 4-(Methylsulphonyl)phenyl bis(2',3'-didehydro-2',3'-dideoxy cytidin-5'-yl)phosphate

N⁴-acetyl-2',3'-didehydro-2',3'-deoxycytidine: 2',3'-didehydro-2',3'-dideoxycytidine (36.2 mmol) is suspended into dry methanol (10000ml) and heated to reflux. Dry acetic anhydride (10 ml, 106 mmol) is added 4 times at every hour (total amount 40 ml, 0.42 mol). The reaction mixture is finally stirred for 6 hr. at refluxed temperature and then left overnight at room temperature. The precipitated crystal is filtered out and washed with ethanol. (73% yield).

### 4-(Methylthio)phenyl bis(N⁴-acetyl-2',3'-didehydro-2',3'-dideoxycytidin-5'-yl)phosphate

4-(methylthio)phenyl phosphorodichloridate, dry 1-methylimidazole and dry acetonitrile are stirred vigorously for 5 min. and then added to a solution of N⁴-acetyl-2',3'-didehydro'2',3'-dideoxycytidine in acetonitrile. After stirring for several hours at room temperature, phosphate buffer is added (pH 6.0) and the mixture is extracted with chloroform. The chloroform extracts are washed with water and then dried over magnesium sulphate. The chloroform is evaporated under reduced pressure and the residue is applied, pre-absorbed, onto silica gel, to a short silica gel column. The column is eluted and the resulting material is further purified using a chromatotron (2 mm plate). The product is then isolated.

### 4-(Methylthio)phenyl bis(2',3'-didehydro-2',3'-dideoxy-cytidin-5'-yl)phosphate

4-(Methylthio)phenyl bis(N⁴-acetyl-2',3'-didehydro-2',3'-dideoxycytidin-5'-yl)phosphate is stirred with potassium carbonate/methanol solution for 20 hrs. at room temperature. After this period the solvent is evaporated under reduced pressure and the residue is applied, pre-absorbed, onto silica gel, to a short silica gel column. The column is eluted and the product is isolated.

### 4-(Methylsulphonyl)phenyl bis(2',3'-didehydro-2',3'-dideoxycytidin-5'-yl)phosphate

4-(Methylthio)phenyl bis(2',3'-didehydro-2',3'-dideoxycytidin-5'-yl)phosphate is dissolved in dry ethanol and cooled to 0°C. A solution of 3-chloroperoxybenzoic acid is added dropwise with stirring over 10 min. and the mixture is stored for 15 hrs. at 5°C. After this period, TLC shows complete conversion of starting material to a major component together with a minor impurity. The solvent is removed by evaporation under reduced pressure and the residue is applied to a 2 mm chromatotron plate in a small volume of chloroform, and then eluted. This purification step is repeated and the product is isolated.

### Example 4

### 4-(Methylsulphonyl)phenyl bis(2',3'-didehydro-2',3'-dideoxyadenosin-5'-yl)phosphate

4-(Methylsulphonyl)phenyl phosphorodichloridate (86 mg, 0.3 mmol), dry 1-methlyimidazole (0.13 ml, 1.4 mmol) and dry pyridine (20 ml) are stirred for 5 min. and then added to 2',3'-didehydro-2',3'-dideoxyadenosine (100 mg, 0.4 mmol). The reaction mixture is stirred vigorously for 18 hrs. at room temperature. T.l.c. shows ca. 50% conversion of starting material to a slower-moving component. A further portion of "phosphorylating agent" (86 mg, 0.3 mmol and 0.13 ml, 1.4 mmol 1-methylimidazole) is added and the reaction is again stirred for 24 hours. After this period, t,l,c shows still ca. 50% conversion to the slower-moving component. The reaction mixture is evaporated to dryness under reduced pressure. The residue is applied, pre-absorbed onto silica gel, to a silica gel column and eluted with chloroform:methanol (10:1). The required fractions are collected and evaporated to dryness, and then dissolved in a minimum of chloroform and triturated with addition of hexane to give the product (23 mg, 16% yield).
NMR Spectrum: δ(d₆DMSO) 3.19(3 H, s, SO₂CH₃), 4.24 (2 H, s, 2x H-5'), 5.04 (2 H, s, 2x H-4'), 6.26 (2 H, t, 2x H-1'), 6.42 (2 H, s, 2x H-3', 6.96 (2 H, s, 2x H-2'), 7.32 (4 H, d, 2x NH₂), 7.22-7.77 (4 H, dd, phenyl), 8.07 (2 H, d, 2x h-2), 8.16 (2 H, s, 2x H-8)
FAB Mass Spectrum: m/z 683 [M + H]⁺

### Example 5

### 4-(Methylsulphonyl)phenyl bis(2',3'-dideoxyadenosin-5'-yl) phosphate

4-(Methylsulphonyl)phenyl phosphorodichloridate (87 mg, 0.3 mmol), dry 1-methylimidazole (0.13 ml, 1.4 and dry pyridine (20 ml) are stirred for 5 min. and then added to 2',3'-dideoxyadenosine (120 mg, 0.5 mmol). The reaction mixture is stirred for 16 hours at room temperature under a stream of nitrogen. T.l.c. shows ca. 40% conversion of starting material to a slower-moving component. A further portion of "phosphorylating agent" (87 mg, 0.3 mmol and 0.13 ml, 0.3 mmol 1-methylimidazole) is added and the reaction mixture is again stirred for 24 hours. The reaction mixture is evaporated to dryness under reduced pressure. The residue is applied, pre-absorbed onto silica gel, to a silica gel column and chromatographed, eluting with dichloromethane:methanol (20:3).
NMR Spectrum: δ(d₆DMSO) 2.08 (4 H, m, 2x H-2'), 2.80 (4 H, m, 2x H-3'), 3.92 (4 H, m, 2x H-5'), 4.26 (2 H, s, 2x H-4'), 6.24 (2 H, m, 2x H-1'), 7.26 (4 H, s, 2x NH₂), 7.31-7.84 (4 H, m, phenyl), 8.13 (2 H, s, 2x H-2), 8.27 (2 H, s, 2x H-8).
FAB Mass Spectrum: m/z 687 [M + H]⁺

### Example 6

### 4-(Methylsulphonyl)phenyl bis(2',3'-didehydro-3'-deoxythymidin-5'-yl)phosphate

4-(Methylsulphonyl)phenylphosphodichloridate (58 mg, 0.2 mmol), dry 1-methylimidazole (85 »l, 1.0 mmol) and dry acetonitrile (5 ml) are stirred vigorously for 5 min. and then added to a solution of 2',3'-didehydro-3'-deoxythymidine (70 mg, 0.3 mmol) in dry acetonitrile (5 ml). After stirring for 17 hours at room temperature under a stream of nitrogen t.l.c. (chloroform:methanol=10:1) shows ca. 60% conversion to a slower-moving component. A further portion of "phosphorylating agent" (22 mg, 0.1 mmol and 0.4 ml, 0.4 mmol 1-methylimidazole) is added, and after stirring for 26 hrs., a further portion of "phosphorylating agent" (13 mg, 0.05 mmol and 0.4 ml, 0.4 mmol 1-methylimidazole) is added. The reaction mixture is stirred at 37°C for 18 hours, but t.l.c. shows the conversion of 60% is not improved at all. After addition of phosphate buffer (20 ml, pH 6.0), the mixture is extracted with chloroform. The organic layer is dried (magnesium sulphate), then evaporated to dryness under reduced pressure. The residue is purified by silica gel column chromatography with ether:methanol (5:1) as eluent to give the product (35 mg, 34% yield).
NMR Spectrum: δ(d₆DMSO) 1.65 (6 H, d, 2x CH₃), 3.25 (3 H, s SO₂CH₃), 4.35 (4 H, m, 2x H-5'), 4.95 (2 H, s, 2x H-4'), 6.05 (2H, m, 2x H-3'), 6.40 (2 H, m, 2x H-2'), 6.85 (2 H, s, 2x H-1'), 7.25-7.40 (4 H, dd, phenyl), 7.90 (2 H, m, 2x H-6), 11.35 (2 H, d, 2x NH).
FAB Mass Spectrum: m/z 665 [M + H]⁺

| Elemental Analysis: | | | |
|---|---|---|---|
| Found: | C, 48.9 ; | H, 4.6 ; | N, 8.5 |
| C₂₇H₂₉O₁₂N₄PS requires | C, 48.8 ; | H, 4.4 ; | N, 8.4%. |

## Claims (Claims for the following Contracting State(s): AT, BE, CH, LI, DE, DK, FR, IT, LU, NL, SE)

1. A compound of formula (I): 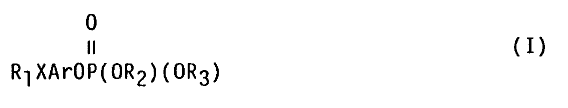 in which formula R₁ represents an alkyl group, Ar represents a substituted or unsubstituted phenylene group, X represents -SO₂- or -CO- and R₂ and R₃ which may be identical or different represent moieties of formula (a), (b), (c), (d), (e), (f), (g), (h) or (i): 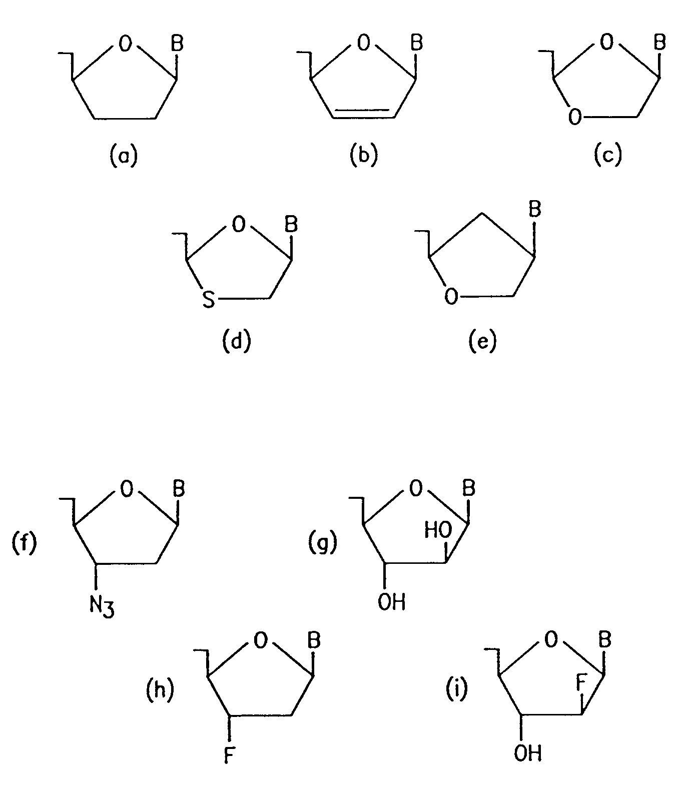 wherein B represents the residue of a nucleoside base of formula (A), (G), (C), (H) or (T): 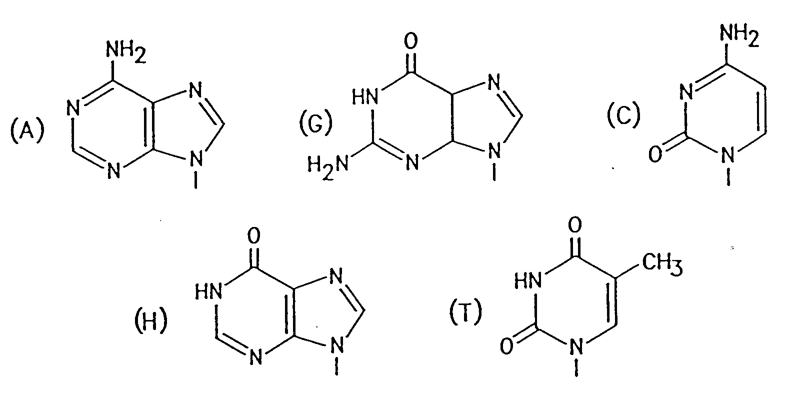 provided that when R₂ and R₃ both represent the moiety of formula (a) then B represents the residue of a nucleoside base which is of formula (A), (G), (C) or (H), the compound optionally being in the form of a pharmaceutically acceptable salt.

2. A compound according to Claim 1, in which Ar represents a phenylene group optionally carrying one or more substituents which may be identical or different and which are halogen, fluoroalkyl, alkoxy, fluoroalkoxy, carbalkoxy, amino or amido.

3. A compound according to Claim 2, in which the one or more substituents are halogen, C₁₋₄ alkoxy, C₁₋₄ fluoroalkoxy, C₁₋₆ carboalkoxy, amino or amido.

4. A compound according to Claim 1, in which Ar represents an unsubstituted phenylene group.

5. A compound according to any of Claims 1 to 4, in which the group R₁X is para to the group OP(O)(OR₂)(OR₃).

6. A compound according to any of Claims 1 to 5, in which R₁ represents a C₁₋₆ alkyl group.

7. A compound according to Claim 6, in which R₁ represents a methyl group.

8. A compound according to any of the preceding claims, in which X represents a sulphonyl group.

9. A compound according to Claim 1, in which R₁XAr represents a 4-(methylsulphonyl)phenyl group.

10. A compound according to any of the preceding claims, in which R₂ and R₃ independently represent moieties of formula (a), (b), (d), or (f).

11. A compound according to Claim 10, in which R₂ and R₃ each represent the moiety of formula (f).

12. A compound according to any of Claims 1 to 9, in which R₂ and R₃ independently have any of the formulae (i) to (x):

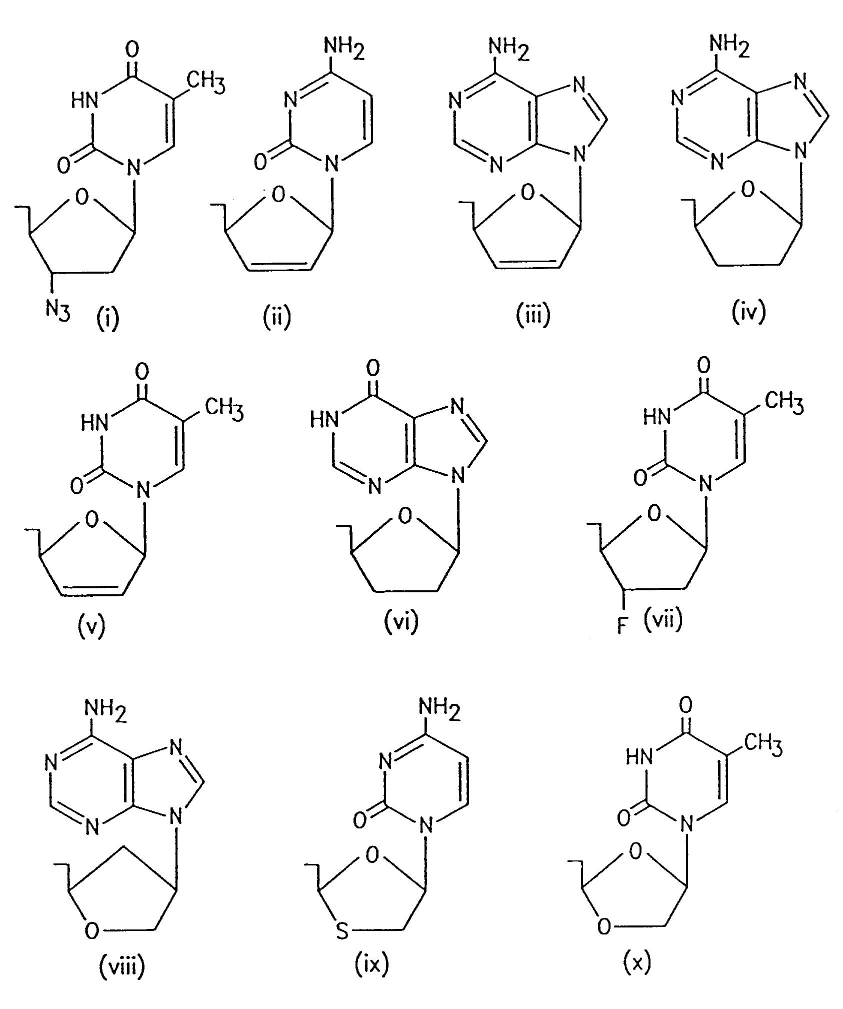

13. A compound according to Claim 12, in which R₂ and R₃ independently have the formula (i), (v), (vi) or (ix).

14. A compound according to Claim 13, in which R₂ and R₃ each has the formula (i).

15. A compound according to Claim 13, in which R₂ and R₃ each has the formula (v).

16. 4-(Methylsulphonyl)phenyl bis(3'-azidothymidin-5'-yl)phosphate.

17. 4-(Methylsulphonyl)phenyl bis(2',3'-didehydro-2',3'-dideoxycytidin-5'-yl)phosphate.

18. 4-(Methylsulphonyl)phenyl bis(2',3'-didehydro-2',3'-dideoxyadenosin-5'-yl)phosphate.

19. 4-(Methylsulphonyl)phenyl bis(2',3'-dideoxyadenosin-5'-yl) phosphate.

20. 4-Methylsulphonyl)phenyl bis(2',3'-didehydro-3'-deoxythymidin-5'-yl)phosphate.

21. A process for the production of a compound of formula (I) according to Claim 1 in which R₂ and R₃ are identical, which comprises reacting a phosphorodihalidate of formula (III), R₁XArOP(O)Y₂, in which R₁, X and Ar are as defined in Claim 1 and Y represents halogen, with a compound of formula R₂OH in which R₂ corresponds to the identical groups R₂ and R₃ in the compound of formula (I) or is a derivative thereof in which a group in the nucleoside base is protected, and thereafter where appropriate removing the protecting group.

22. A process according to Claim 21, in which the nucleoside base in R₂ is cytosine protected by acetylation.

23. A process according to Claim 21 or 22, in which the reaction is conducted in the presence of a base.

24. A process according to Claim 23, in which the base is 1-methylimidazole.

25. A process for the production of a compound of formula (I) according to Claim 1 having a group X which represents -SO₂- in which a compound of formula (IV), R₁SArOP(O)(OR₂)(OR₃), or a compound of formula (V), R₁SOArOP(O)(OR₂)(OR₃), in which formulae R₁, Ar, R₂ and R₃ are as defined in Claim 1, is oxidized to convert the group -S- or -SO-, respectively, to a group -SO₂-.

26. A process according to Claim 25 in which a per acid is used to oxidize the compound of formula (IV) or (V).

27. A compound of formula (IV), R₁SArOP(O)(OR₂)(OR₃), in which R₁, Ar, R₂ and R₃ are as defined in Claim 1.

28. A compound of formula (V), R₁SOArOP(O)(OR₂)(OR₃), in which R₁, Ar, R₂ and R₃ are as defined in Claim 1.

29. A compound of formula (I) according to any of Claims 1 to 20 for use in therapy.

30. The use of a compound of formula (I) according to any of Claims 1 to 20 in the manufacture of a medicament for use in the treatment or prophylaxis of a human retrovirus infection.

31. The use according to Claim 30, in which the virus is Human Immunodeficiency Virus (HIV).

32. A method for the treatment of blood to inhibit or prevent viral replication therein of a human retrovirus which comprises treating extra-corporeal blood with a compound of formula (I) as defined in Claim 1.

33. A method according to claim 32, in which the virus is Human Immunodeficiency Virus (HIV).

34. A pharmaceutical composition which comprises a compound of formula (I) as defined in any of Claims 1 to 20 together with a pharmaceutically acceptable carrier therefor.

35. A pharmaceutical composition according to Claim 34, in dosage form.

## Claims (Claims for the following Contracting State(s): ES)

1. A process for the production of a compound of formula (I) 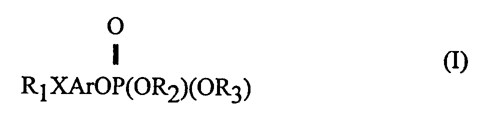 in which formula R₁ represents an alkyl group,
Ar represents a substituted or unsubstituted phenylene group,
X represents -SO₂- or -CO- and R₂ and R₃ which are identical represent moieties of formula (a), (b), (c), (d), (e), (f), (g), (h) or (i): 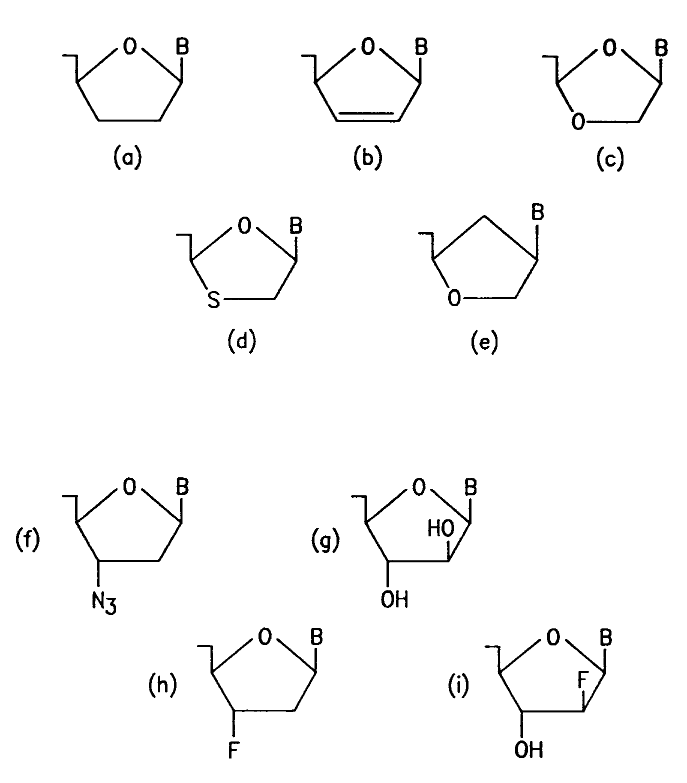 wherein B represents the residue of a nucleoside base of formula (A), (G), (C), (H) or (T): 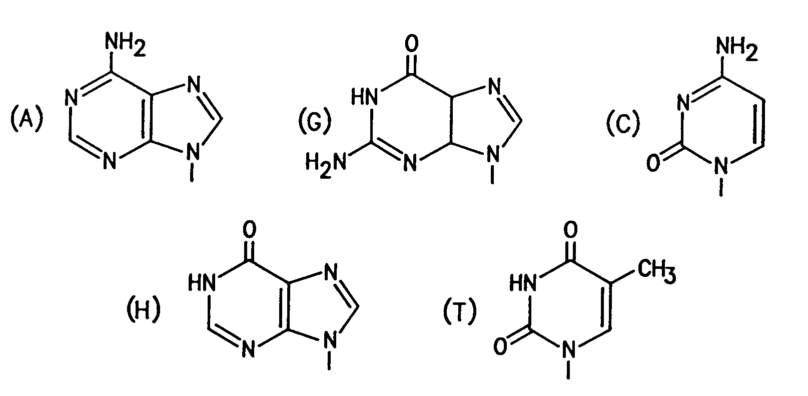 provided that when R₂ and R₃ both represent the moiety of formula (a) then B represents the residue of a nucleoside base which is of formula (A), (G), (C) or (H), the compound optionally being in the form of a pharmaceutically acceptable salt, which comprises reacting a phosphorodihalidate of formula (III), R₁XArOP(O)Y₂, in which R₁, X and Ar are as defined for the compound of formula (I) and Y represents halogen, with a compound of formula R₂OH in which R₂ corresponds to the identical groups R₂ and R₃ in the compound of formula (I) or is a derivative thereof in which a group in the nucleoside base is protected, ad thereafter where appropriate removing the protecting group.

2. A process according to Claim 1, in which the nucleoside base in R₂OH is protect by acetylation when R₂ is cytosine.

3. A process according to Claim 1 or 2, in which the reaction is conducted in the presence of a base.

4. A process according to Claim 3, in which the base is 1-methylimidazole.

5. A process according to any of Claims 1 to 4, in which X represents a sulphonyl group.

6. A process for the production of a compound of formula (I) 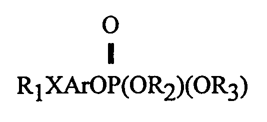 in which formula R₁ represents an alkyl group,
Ar represents a substituted or unsubstituted phenylene group,
X represents -SO₂- and R₂ and R₃ which may be identical or different represent moieties of formula (a), (b), (c), (d), (e), (f), (g), (h) or (i): 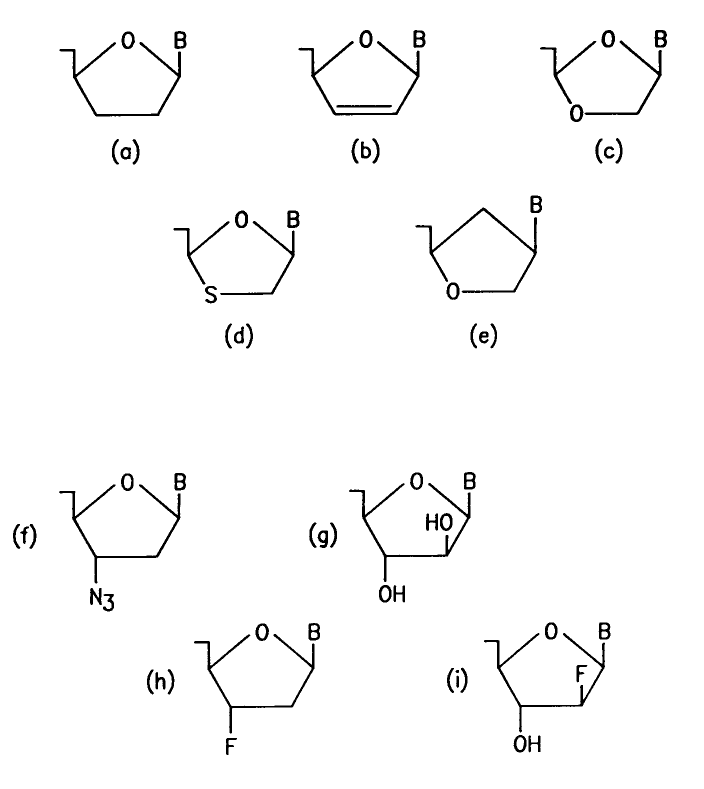 wherein B represents the residue of a nucleoside base of formula (A), (G), (C), (H) or (T): 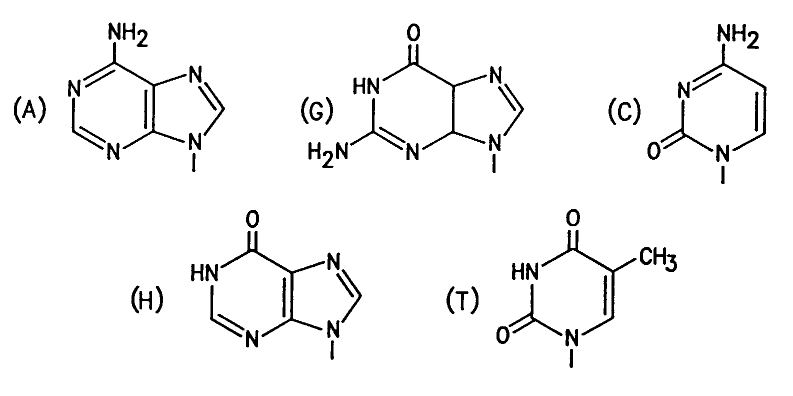 provided that when R₂ and R₃ both represent the moiety of formula (a) then B represents the residue of a nucleoside base which is of formula (A), (G), (C) or (H), the compound optionally being in the form of a pharmaceutically acceptable salt, which comprises oxidizing a compound of formula (IV), R₁ SArOP(O)(OR₂)(OR₃), or a compound of formula (V), R₁ SOArOP(O)(OR₂)(OR₃), in which formulae R₁, Ar, R₂ and R₃ are as defined for the compound of formula (I), to convert the group -S- or -SO-, respectively, to a group -SO₂-.

7. A process according to Claim 6, in which a per acid is used to oxidize the compound of formula (IV) or (V).

8. A process according to any of Claims 1 to 7, in which Ar represents a phenylene group optionally carrying one or more substituents which may be identical or different and which are halogen, fluoroalkyl, alkoxy, fluoroalkoxy, carbalkoxy, amino or amido.

9. A process according to Claim 8, in which the one or or more substituents are halogen, C₁₋₄ alkoxy, C₁₋₄ fluoroalkoxy, C₁₋₆ carboalkoxy, amino or amido.

10. A process according to any of Claims 1 to 7, in which Ar represents an unsubstituted phenylene group.

11. A process according to any of the preceding claims, in which the group R₁X is para to the group OP(O)(OR₂)(OR₃).

12. A process according to any of the preceding claims, in which R₁ represents a C₁₋₆ alkyl group.

13. A process according to Claim 12, in which R₁ represents a methyl group.

14. A process according to any of Claims 1 to 6, in which R₁XAr represents a 4-(methylsulphonyl)phenyl group.

15. A process according to any of the preceding claims, in which R₂ and R₃ either each, or independently or each, represent moieties of formula (a), (b), (d) or (f).

16. A process according to Claim 15, in which R₂ and R₃ each represent the moiety of formula (f).

17. A process according to any of Claims 1 to 14, in which R₂ and R₃ either each, or independently or each, have any of the formulae (i) to (x):

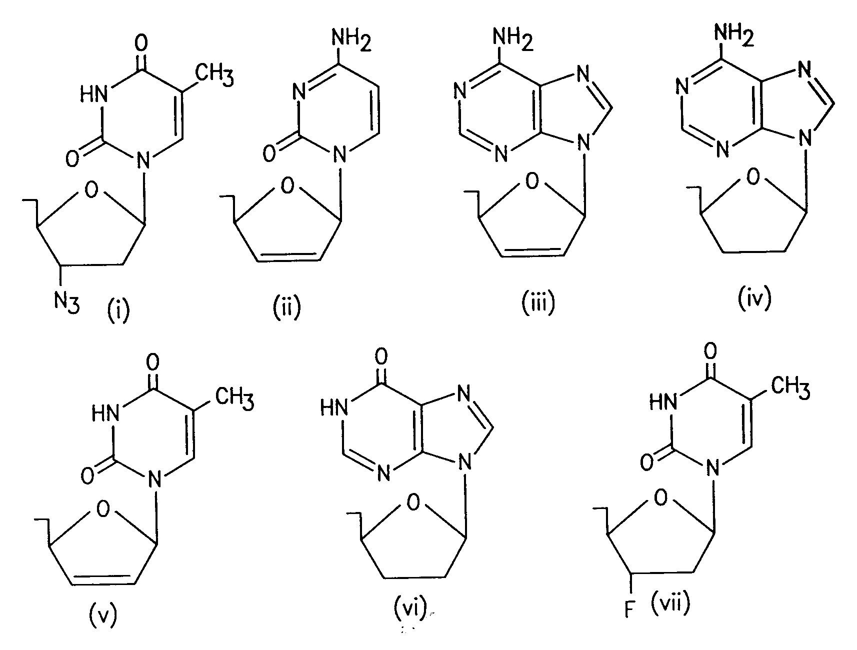

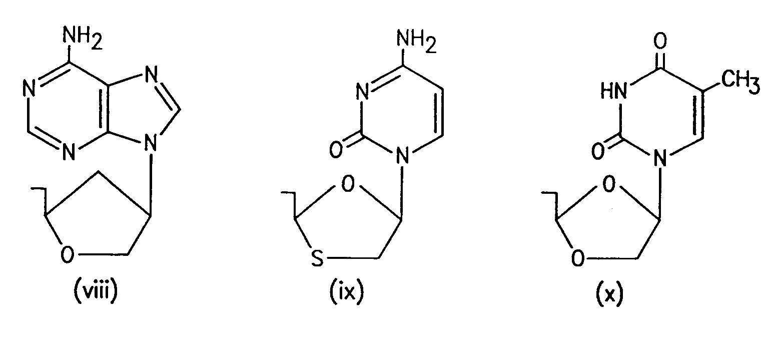

18. A process according to Claim 17, in which R₂ and R₃ either each, or independently or each, have the formula (i), (v), (vi) or (ix).

19. A process according to Claim 18, in which R₂ and R₃ each has the formula (i).

20. A process according to Claim 18, in which R₂ and R₃ each has the formula (v).

21. A process according to any of Claims 1 to 7, in which the compound of formula (I) is 4-(methylsulphonyl)phenyl bis(3'-azidothymidin-5'-yl)phosphate.

22. A process according to any of Claims 1 to 7, in which the compound of formula (I) is 4-(methylsulphonyl)phenyl bis(2',3'-didehydro-2',3'-dideoxycytidin-5'-yl)phosphate.

23. A process according to any of Claims 1 to 7, in which the compound of formula (I) is 4-(methylsulphonyl)phenyl bis(2',3'-didehydro-2',3'-dideoxyadenosin-5'-yl)phosphate.

24. A process according to any of Claims 1 to 7, in which the compound of formula (I) is 4-(methylsulphonyl)phenyl bis(2',3'-dideoxyadenosin-5'-yl)phosphate.

25. A process according to any of Claims 1 to 7, in which the compound of formula (I) is 4-(methylsulphonyl)phenyl bis(2',3'didehydro-3'-deoxythymidin-5'-yl)phosphate.

## Patentansprüche (Patentansprüche für folgende(n) Vertragsstaat(en): AT, BE, CH, LI, DE, DK, FR, IT, LU, NL, SE)

1. Eine Verbindung der Formel (I): 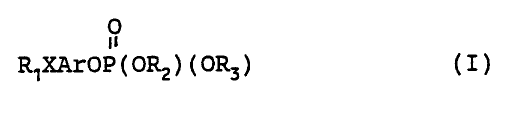 in welcher Formel R₁ eine Alkylgruppe darstellt,
Ar eine substituierte oder unsubstituierte Phenylengruppe darstellt,
X -SO₂- oder -CO- darstellt und
R₂ und R₃, welche identisch oder verschieden sein können, Komponenten der Formel (a), (b), (c), (d) (e), (f), (g), (h) oder (i) darstellen: 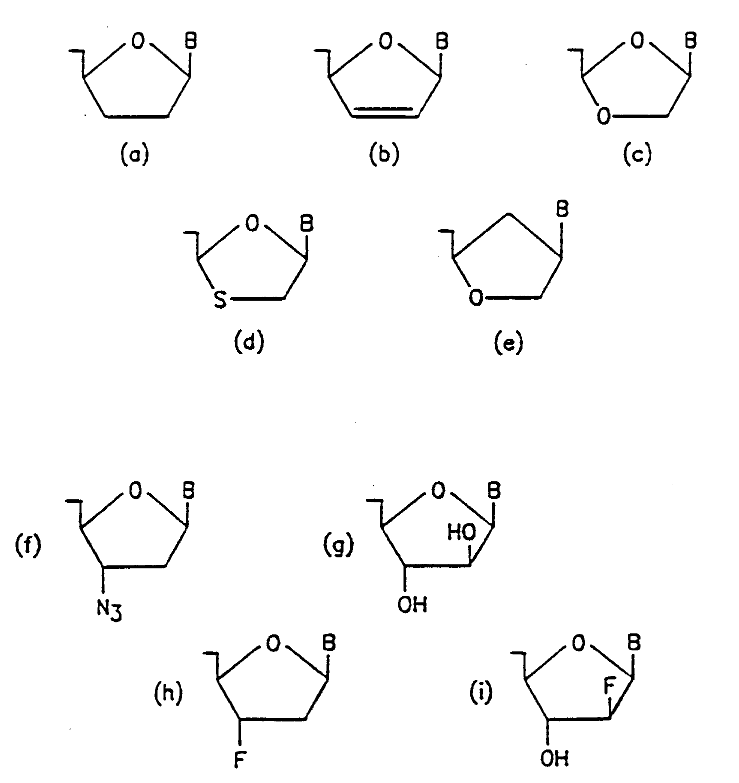 worin B den Rest einer Nucleosidbase der Formel (A), (G), (C), (H) oder (T) darstellt: 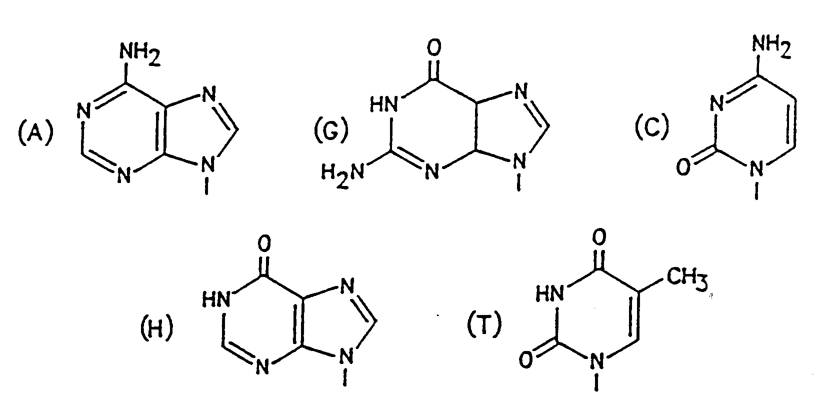 mit der Maßgabe, daß wenn sowohl R₂ als auch R₃ die Komponente der Formel (a) darstellen, B dann den Rest einer Nucleosidbase darstellt, welche der Formel (A), (G), (C) oder (H) entspricht, wobei die Verbindung wahlweise in Form eines pharmazeutisch annehmbaren Salzes vorliegt.

2. Verbindung nach Anspruch 1, in der Ar eine Phenylengruppe darstellt, welche wahlweise einen oder mehrere Substituenten trägt, die identisch oder verschieden sein können, und welche Halogen-, Fluoralkyl-, Alkoxy-, Fluoralkoxy-, Carbalkoxy-, Amino- oder Amido-Substituenten sind.

3. Verbindung nach Anspruch 2, in welcher der eine oder die mehreren Substituenten Halogen-, C₁₋₄-Alkoxy-, C₁₋₄-Fluoralkoxy, C₁₋₆-Carbalkoxy-, Amino- oder Amidosubstituenten sind.

4. Verbindung nach Anspruch 1, in welcher Ar eine unsubstituierte Phenylengruppe darstellt.

5. Verbindung nach jedem der Ansprüche 1 bis 4, in welcher sich die Gruppe R₁X in Para-Stellung zur Gruppe OP(O)(OR₂)(OR₃) befindet.

6. Verbindung nach jedem der Ansprüche 1 bis 5, in der R₁ eine C₁₋₆-Alkylgruppe darstellt.

7. Verbindung nach Anspruch 6, in welcher R₁ eine Methylgruppe darstellt.

8. Verbindung nach jedem der vorhergehenden Ansprüche, in welcher X eine Sulfonylgruppe darstellt.

9. Verbindung nach Anspruch 1, in welcher R₁XAr eine 4-(Methylsulfonyl)phenylgruppe darstellt.

10. Verbindung nach jedem der vorhergehenden Ansprüche, in welcher R₂ und R₃ unabhängig voneinander Komponenten der Formel (a), (b), (d) oder (f) darstellen.

11. Verbindung nach Anspruch 10, in welcher R₂ und R₃ jeweils die Komponente von Formel (f) darstellen.

12. Verbindung nach jedem der Ansprüche 1 bis 9, in welcher R₂ und R₃ unabhängig voneinander jede der Formeln (i) bis (x) aufweisen:

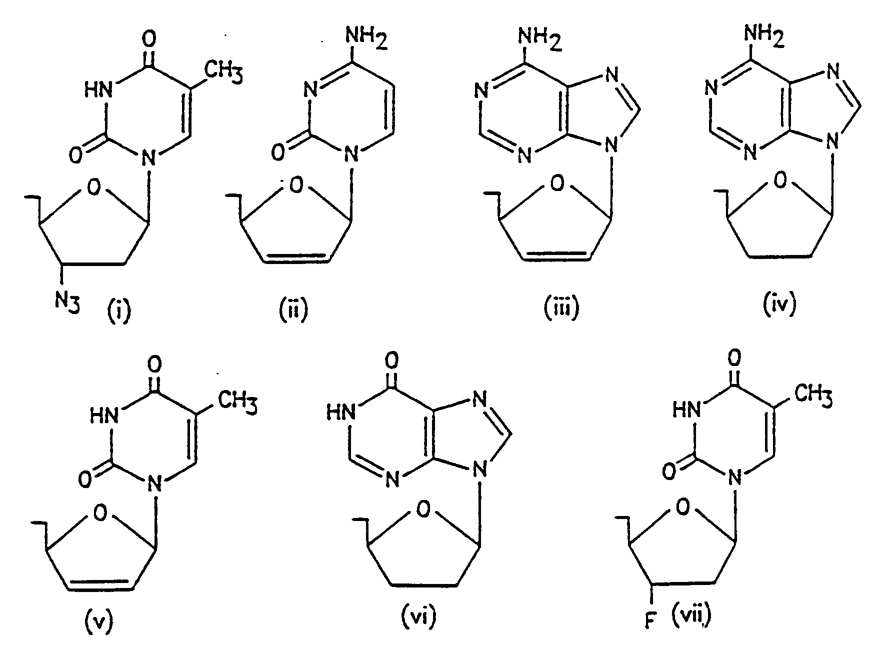

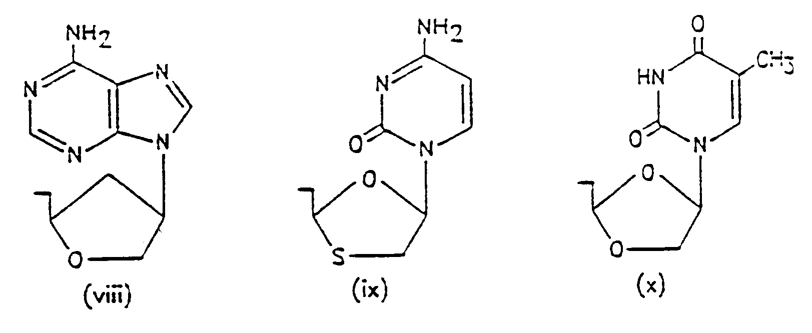

13. Verbindung nach Anspruch 12, in welcher R₂ und R₃ unabhängig voneinander die Formel (i), (v), (vi) oder (ix) darstellen.

14. Verbindung nach Anspruch 13, in welcher R₂ und R₃ jeweils die Formel (i) entsprechen.

15. Verbindung nach Anspruch 13, in welcher R₂ und R₃ jeweils die Formel (v) besitzen.

16. 4-(Methylsulfonyl)phenyl-bis-(3'-azidothymidin-5'-yl)-phosphat.

17. 4-(Methylsulfonyl)phenyl-bis-(2,3'didehydro-2',3'-dideoxycytidin-5'-yl)-phosphat.

18. 4-(Methylsulfonyl)phenyl-bis-(2',3'-didehydro-2',3-dideoxy-adenosin-5'-yl)-phosphat.

19. 4-(Methylsulfonyl)phenyl-bis-(2',3'-dideoxy-adenosin-5'-yl)-phosphat.

20. 4-(Methylsulfonyl)phenyl-bis-(2',3'-didehydro-3'-deoxythymidin-5'-yl)-phosphat.

21. Ein Verfahren zur Herstellung einer Verbindung der Formel (I) gemäß Anspruch 1, in welcher R₂ und R₃ identisch sind, welches die Umsetzung eines Phosphordihalogenids der Formel (III), R₁XArOP(O)Y₂, in welcher R₁, X und Ar wie in Anspruch 1 definiert sind und Y einen Halogenrest darstellt, mit einer Verbindung der Formel R₂OH, in welcher R₂ den identischen Gruppen R₂ und R₃ in der Verbindung der Formel (I) entspricht oder ein Derivat davon ist, in welcher eine Gruppe in der Nucleosidbase geschützt ist und danach, wo dies geeignet ist, Entfernung der Schutz-Gruppe umfaßt.

22. Verfahren nach Anspruch 21, in welchem die Nucleosidbase in R₂ durch Acetylierung geschütztes Cytosin ist.

23. Verfahren nach Anspruch 21 oder 22, in welchem die Reaktion in Gegenwart einer Base durchgeführt wird.

24. Verfahren nach Anspruch 23, in welchem die Base 1-Methylimidazol ist.

25. Ein verfahren zur Herstellung einer Verbindung der Formel (I) gemäß Anspruch 1, welche ein Gruppe X aufweist, die -SO₂-darstellt, in welchem eine Verbindung der Formel (IV), R₁SArOP(O)(OR₂)(OR₃), oder eine Verbindung der Formel (V), R₁SOArOP(O)(OR₂)(OR₃), in welchen Formeln R₁, Ar, R₂ und R₃ wie in Anspruch 1 definiert sind, oxidiert werden, um die Gruppe -S- bzw. -SO- in eine -SO₂-Gruppe umzuwandeln.

26. Verfahren nach Anspruch 25, in welchem eine Persäure verwendet wird, um die Verbindung der Formel (IV) oder (V) zu oxidieren.

27. Eine Verbindung der Formel (IV), R₁SArOP(O)(OR₂)(OR₃), worin R₁, Ar, R₂ und R₃ wie in Anspruch 1 definiert sind.

28. Eine Verbindung der Formel (V) R₁SOArOP(O)(OR₂)(OR₃), worin R₁, Ar, R₂ und R₃ wie in Anspruch 1 definiert sind.

29. Verbindung der Formel (I) gemäß jedem der Ansprüche 1 bis 20 zur Verwendung bei der Therapie.

30. Verwendung der Verbindung der Formel (I) gemäß jedem der Ansprüche 1 bis 20 bei der Herstellung eines Medikamentes zur Verwendung bei der Behandlung oder Vorbeuge einer Retrovirus-Infektion beim Menschen.

31. Verwendung gemäß Anspruch 30, in welcher der Virus ein Immundefekt-Virus (HIV) ist.

32. Ein Verfahren zur Behandlung von Blut, um die virale Replikation darin eines menschlichen Retrovirus darin zu inhibieren oder vorzubeugen, das eine extrakorporale Blutbehandlung mit einer wie in Anspruch 1 definierten Verbindung der Formel (I) umfaßt.

33. Verfahren gemäß Anspruch 32, worin der Virus Immundefekt-Virus HIV ist.

34. Eine pharmazeutische Zusammensetzung, welche eine wie in jedem der Ansprüche 1 bis 20 definierte Verbindung der Formel (I) zusammen mit einem pharmazeutisch annehmbaren Träger dafür umfaßt.

35. Pharmazeutische Zusammensetzung nach Anspruch 34, in Dosisform.

## Patentansprüche (Patentansprüche für folgende(n) Vertragsstaat(en): ES)

1. Ein Verfahren zur Herstellung einer Verbindung der Formel (I): 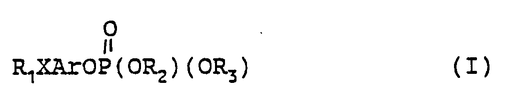 in welcher Formel R₁ eine Alkylgruppe darstellt,
Ar eine substituierte oder unsubstituierte Phenylengruppe darstellt,
X -SO₂- oder -CO- darstellt und
R₂ und R₃, welche identisch sind, Komponenten der Formel (a), (b), (c), (d) (e), (f), (g), (h) oder (i) darstellen: 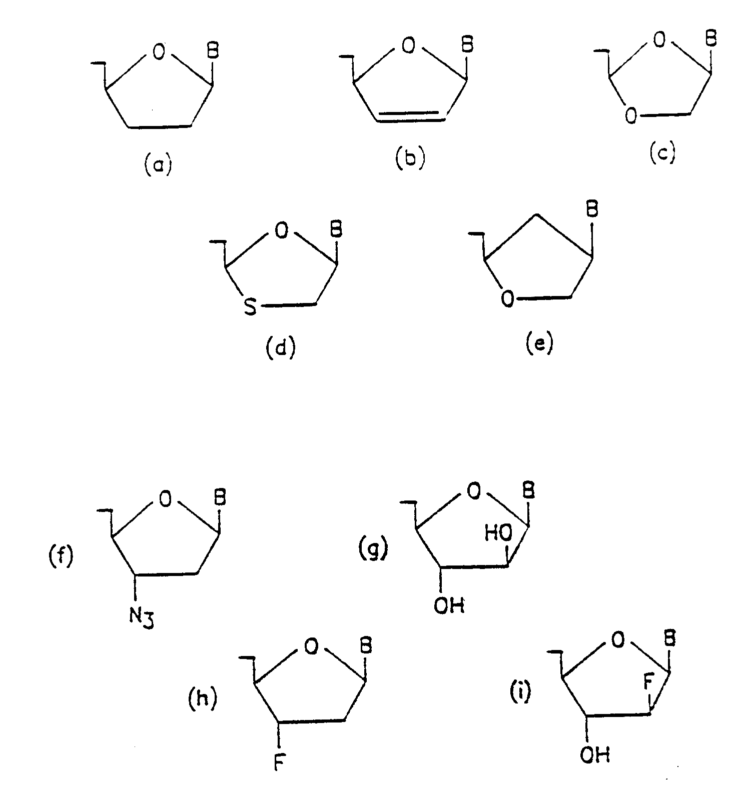 worin B den Rest einer Nucleosidbase der Formel (A), (G), (C), (H) oder (T) darstellt: 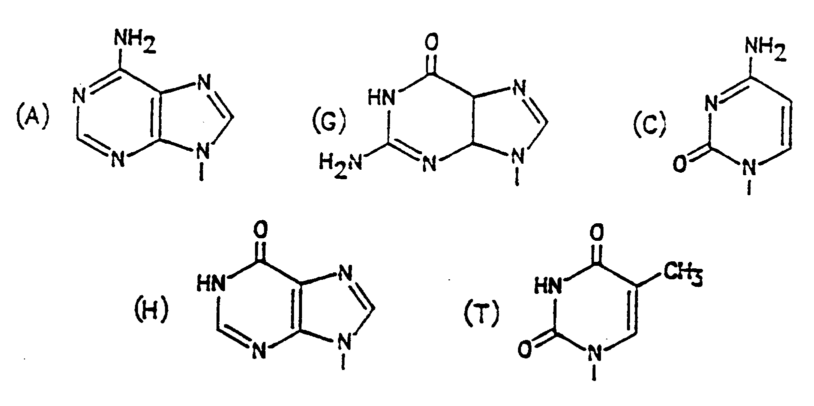 mit der Maßgabe, daß wenn sowohl R₂ als auch R₃ die Gruppe der Formel (a) darstellen, B dann den Rest einer Nucleosidbase darstellt, welche der Formel (A), (G), (C) oder (H) entspricht, wobei die Verbindung wahlweise in Form eines pharmazeutisch annehmbaren Salzes vorliegt, das die Umsetzung eines Phosphordihalogenids der Formel (III), R₁XArOP(O)Y₂, worin R₁, X und Ar die für die Formel (I) angegebene Bedeutung besitzen und Y ein Halogenatom darstellt, mit einer Verbindung der Formel R₂OH, worin R₂ den identischen Gruppen R₂ und R₃ in der Verbindung der Formel (I) entspricht oder ein Derivat davon ist, in welcher eine Gruppe in der Nucleosidbase geschützt ist und danach gegebenenfalls Entfernen der Schutzgruppe umfaßt.

2. Verfahren nach Anspruch 1, worin die Nucleosidbase in R₂OH durch Acetylierung geschützt wird, wenn R₂ Cytosin ist.

3. Verfahren nach Anspruch 1 oder 2, worin die Reaktion in Gegenwart einer Base durchgeführt wird.

4. Verfahren nach Anspruch 3, worin die Base 1-Methylimidazol ist.

5. Verfahren nach jedem der Ansprüche 1 bis 4, worin X eine Sulfonylgruppe darstellt.

6. Ein Verfahren zur Herstellung einer Verbindung der Formel (I) 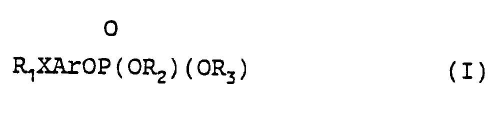 in welcher Formel R₁ eine Alkylgruppe darstellt,
Ar eine substituierte oder unsubstituierte Phenylengruppe darstellt,
X -SO₂- darstellt und R₂ und R₃, welche identisch oder verschieden sein können, Komponenten der Formel (a), (b), (c), (d), (e), (f), (g), (g) oder (i) darstellen: 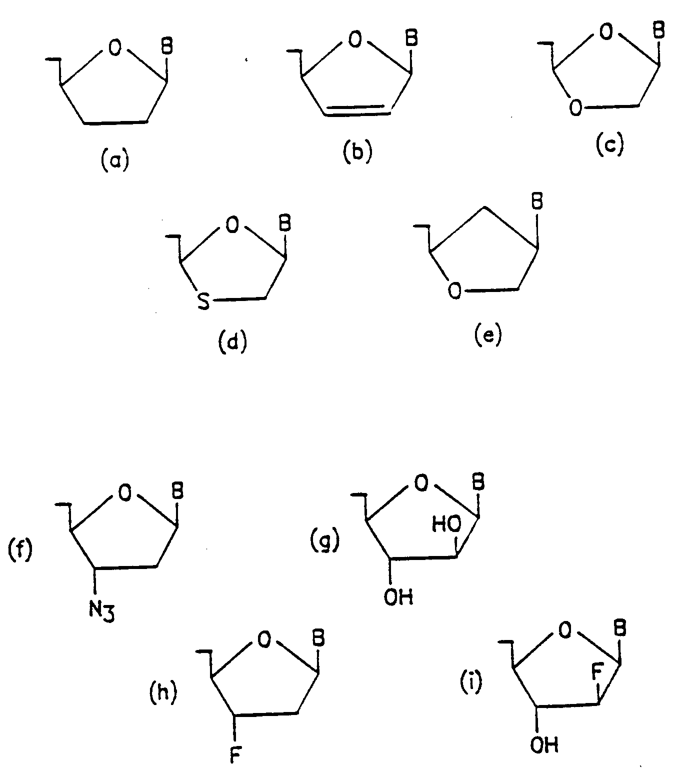 worin B den Rest einer Nucleosidbase der Formel (A), (G), (C), (H) oder (T) darstellt: 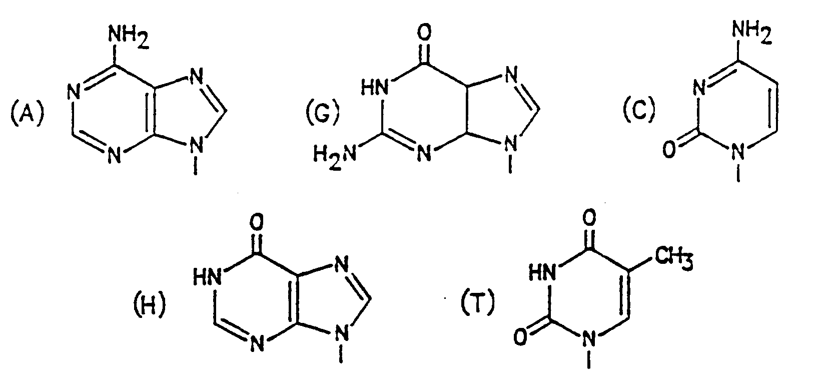 mit der Maßgabe, daß wenn sowohl R₂ als auch R₃ die Gruppe der Formel (a) darstellen; B dann den Rest einer Nucleosidbase darstellt, welche der Formel (A), (G), (C) oder (H) entspricht, wobei die Verbindung wahlweise in Form eines pharmazeutisch annehmbaren Salzes vorliegt, welches die Oxidierung einer Verbindung der Formel (IV), R₁SArOP(O)(OR₂)(OR₃) oder einer Verbindung der Formel (V) R₁SOArOP(O)(OR₂)(OR₃) umfaßt, in welchen Formeln R₁, Ar, R₂ und R₃ die für die Verbindung der Formel (I) angegebene Bedeutung besitzen, um die Gruppe -S- bzw. -SO- in eine -SO₂-Gruppe umzuwandeln.

7. Verfahren nach Anspruch 6, worin eine Persäure verwendet wird, um die Verbindung der Formel (IV) oder (V) zu oxidieren.

8. Verfahren nach jedem der Ansprüche 1 bis 7, worin Ar eine Phenylengruppe darstellt, welche wahlweise eine oder mehrere Substituenten trägt, welche identisch oder verschieden sein können und welche ein Halogen-, Fluoralkyl-, Alkoxy-, Fluoralkoxy-, Carbalkoxy-, Amino- oder Amidosubstituent sind.

9. Verfahren nach Anspruch 8, in welchem der eine oder die mehreren Substituenten Halogen-, C₁₋₄-Alkoxy-, C₁₋₄-Fluoralkoxy-, C₁₋₆-Carbalkoxy-, Amino- oder Amidosubstituenten sind.

10. Verfahren nach jedem der Ansprüche 1 bis 7, worin Ar eine unsubstituierte Phenylengruppe darstellt.

11. Verfahren nach jedem der vorhergehenden Ansprüche, worin die Gruppe R₁X sich in Para-Stellung zur Gruppe OP(O)(OR₂)(OR₃) befindet.

12. Verfahren nach jedem der vorhergehenden Ansprüche, worin R₁ eine C₁₋₆-Alkylgruppe darstellt.

13. Verfahren nach Anspruch 12, worin R₁ eine Methylgruppe darstellt.

14. Verfahren nach jedem der Ansprüche 1 bis 6, worin R₁XAr eine 4-(Methylsulphonyl)phenylgruppe darstellt.

15. Verfahren nach jedem der vorhergehenden Ansprüche, worin R₂ und R₃ entweder jeweils, oder unabhängig oder jeweils Gruppen der der Formel (a), (b), (d) oder (f) darstellen.

16. Verfahren nach Anspruch 15, worin R₂ und R₃ jeweils die Gruppe der Formel (f) darstellen.

17. Verfahren nach jedem der Ansprüche 1 bis 14, worin R₂ und R₃ entweder jeweils, oder unabhängig oder jeweils jede der Formeln (i) bis (x) besitzen:

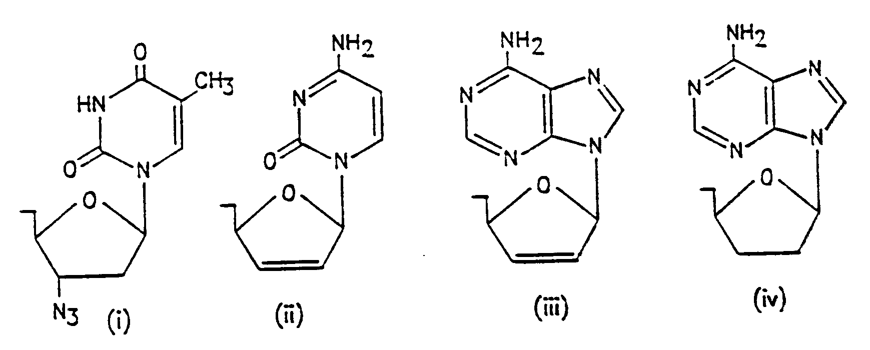

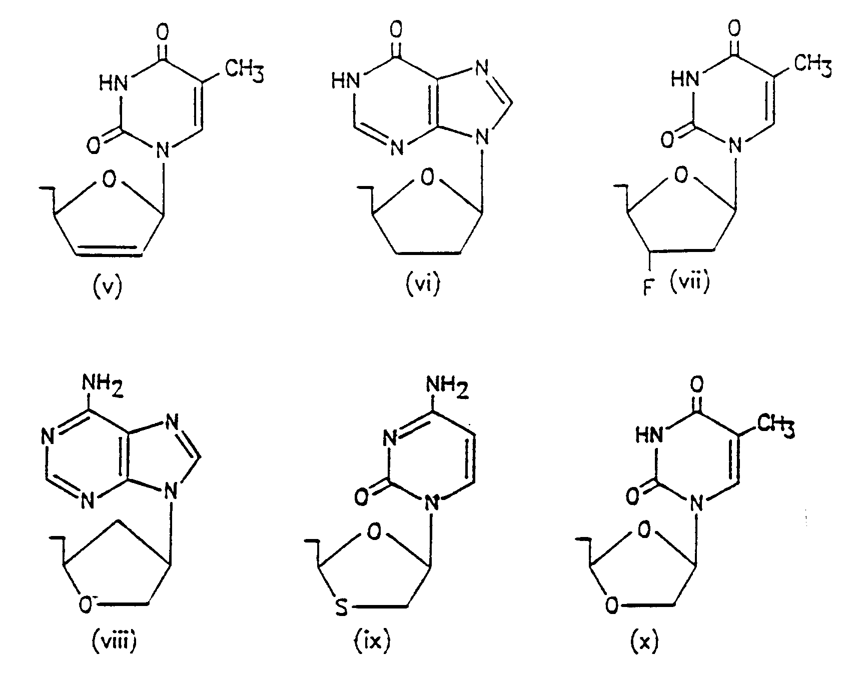

18. Verfahren nach Anspruch 17, worin R₂ und R₃ entweder jeweils, oder unabhängig oder jeweils die Formel (i), (v), (vi) oder (xi) darstellen.

19. Verfahren nach Anspruch 18, worin R₂ und R₃ jeweils die Formel (i) besitzen.

20. Verfahren nach Anspruch 18, worin R₂ und R₃ jeweils die Formel (v) besitzen.

21. Verfahren nach jedem der Ansprüche 1 bis 7, worin die Verbindung der Formel (I) 4-(Methylsulfonyl)phenyl-bis-(3'-azidothymidin-5'-yl)phosphat ist.

22. Verfahren nach jedem der Ansprüche 1 bis 7, worin die Verbindung der Formel (I) 4-(Methylsulfonyl)phenyl-bis-(2,3'-didehydro-2',3,-dideoxcytidin-5'-yl)phosphat ist.

23. Verfahren nach jedem der Ansprüche 1 bis 7, worin die Verbindung der Formel (I) 4-(Methylsulfonyl)phenyl-bis-(2',3'-didehydro-2',3'-dideoxyadenosin-5'-yl)phosphat ist.

24. Verfahren nach jedem der Ansprüche 1 bis 7, worin die Verbindung der Formel (I) 4-(Methylsulfonyl)phenyl-bis-(2',3'-dideoxyadenosin-5'-yl)phosphat ist.

25. Verfahren nach jedem der Ansprüche 1 bis 7, worin die Verbindung der Formel (I) 4-(Methylsulfonyl)phenyl-bis-(2',3'-didehydro-3'-deoxythymidin-5'-yl)phosphat ist.

## Revendications (Revendications pour l'(les) Etat(s) contractant(s) suivant(s): AT, BE, CH, LI, DE, DK, FR, IT, LU, NL, SE)

1. Composé de formule (I) : 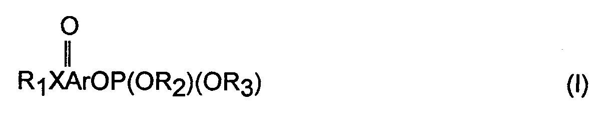 formule dans laquelle R₁ représente un groupe alkyle,
Ar représente un groupe phénylène substitué ou non substitué, X représente -SO₂- ou -CO- et R₂ et R₃ qui peuvent être identiques ou différents représentent des parties de formule (a), (b), (c), (d), (e), (f), (g), (h) ou (i) : 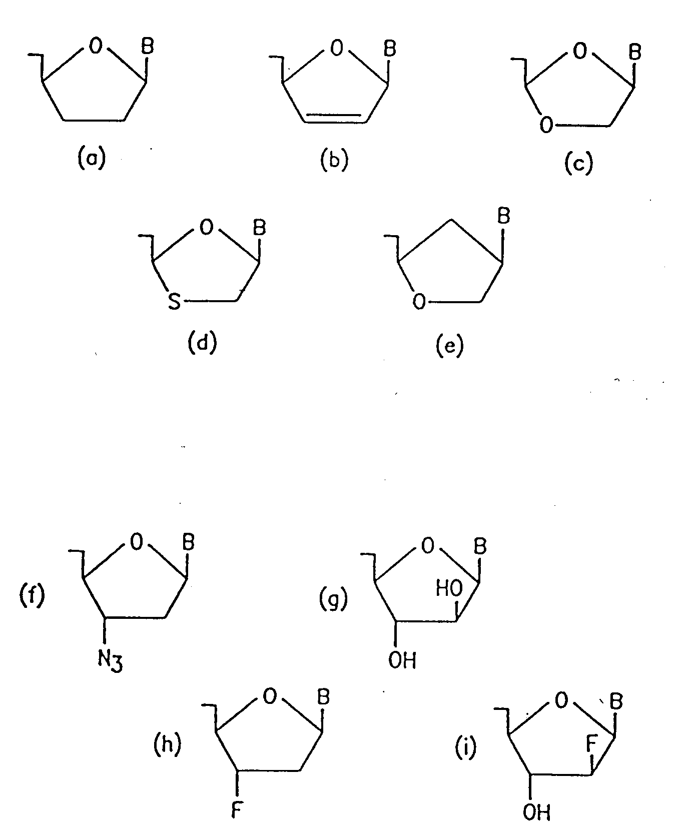 où B représente le résidu d'une base nucléosidique de formule (A), (G), (C), (H) ou (T) : 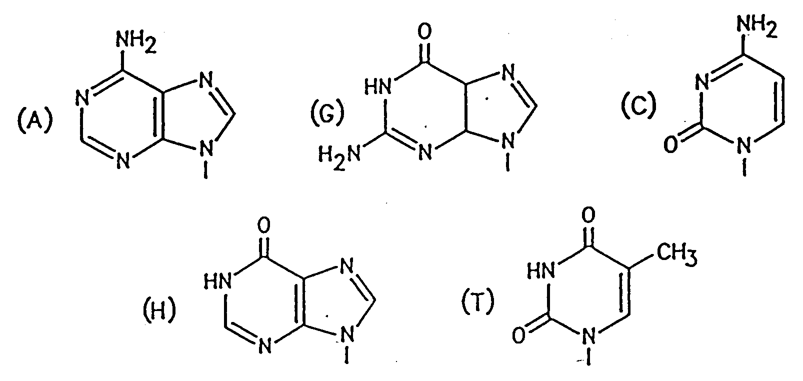 à condition que, lorsque R₂ et R₃ représentent tous les deux la partie de formule (a) alors B représente le résidu d'une base nucléosidique qui est de formule (A), (G), (C) ou (H), le composé étant éventuellement sous la forme d'un sel pharmaceutiquement acceptable.

2. Composé selon la revendication 1, dans lequel Ar représente un groupe phénylène portant éventuellement un ou plusieurs substituants qui peuvent être identiques ou différents et qui sont un atome d'halogène, un groupe fluoroalkyle, alcoxy, fluoroalcoxy, carbalcoxy, amino ou amido.

3. Composé selon la revendication 2, dans lequel un ou plusieurs substituants sont un atome d'halogène, un groupe alcoxy en C₁₋₄, fluoroalcoxy en C₁₋₄, carboalcoxy en C₁₋₆, amino ou amido.

4. Composé selon la revendication 1, dans lequel Ar représente un groupe phénylène non substitué.

5. Composé selon l'une quelconque des revendications 1 à 4, dans lequel le groupe R₁X est en para par rapport au groupe OP(O)(OR₂)(OR₃).

6. Composé selon l'une quelconque des revendications 1 à 5, dans lequel R₁ représente un groupe alkyle en C₁-₆.

7. Composé selon la revendication 6, dans lequel R₁ représente un groupe méthyle.

8. Composé selon l'une quelconque des revendications précédentes, dans lequel X représente un groupe sulfonyle.

9. Composé selon la revendication 1, dans lequel R₁XAr représente un groupe 4-(méthylsulfonyl)phényle.

10. Composé selon l'une quelconque des revendications précédentes, dans lequel R₂ et R₃ représentent, indépendamment, des parties de formule (a), (b), (d), ou (f).

11. Composé selon la revendication 10, dans lequel R₂ et R₃ représentent chacun la partie de formule (f).

12. Composé selon l'une quelconque des revendications 1 à 9, dans lequel R₂ et R₃ ont indépendamment l'une quelconque des formules (i) à (x) :

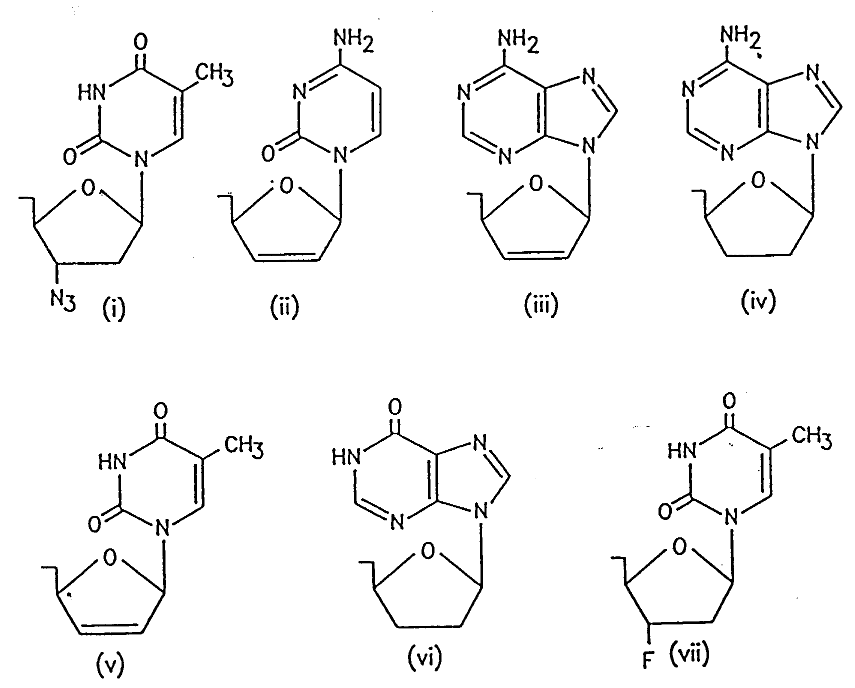

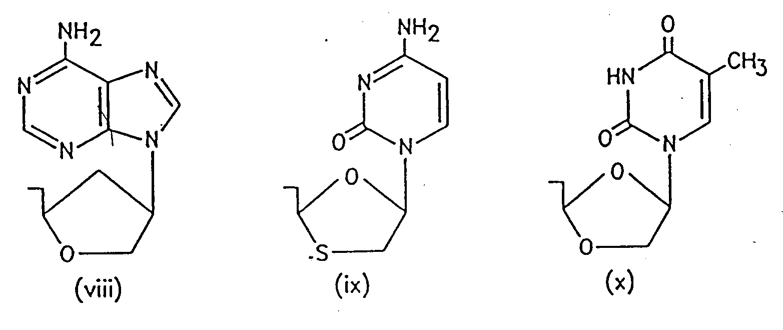

13. Composé selon la revendication 12, dans lequel R₂ et R₃ ont indépendamment la formule (i), (v), (vi) ou (ix).

14. Compose selon la revendication 13, dans lequel R₂ et R₃ ont chacun la formule (i).

15. Composé selon la revendication 13, dans lequel R₂ et R₃ ont chacun la formule (v).

16. 4-(Méthylsulfonyl)phényl bis(3'-azidothymidine-5'-yl)phosphate.

17. 4-(Méthylsulfonyl)phényl bis(2',3'-didéshydro-2',3'-didésoxycytidine-5'-yl) phosphate.

18. 4-(Méthylsulfonyl)phényl bis(2',3'-didéshydro-2',3' didésoxyadénosine-5'-yl) phosphate.

19. 4-(Méthylsulfonyl)phényl bis(2',3'-didésoxyadénosine-5'-yl)phosphate.

20. 4-(Méthylsulfonyl)phényl bis(2',3'-didéshydro-3'-désoxythymidine-5'-yl)phosphate.

21. Procédé de production d'un composé de formule (I) selon la revendication 1 dans lequel R₂ et R₃ sont identiques, qui comprend une étape consistant à faire réagir un phosphonodihalogénure de formule (III), R₁XArOP(O)Y₂, dans laquelle R₁, X et Ar sont tels que défnis à la revendication 1 et Y représente un atome d'halogène, avec un composé de formule R₂OH dans laquelle R₂ correspond aux groupes identiques R₂ et R₃ dans le composé de formule (I) ou est un dérivé de celui-a dans lequel un groupe de la base nucléosidique est protégé, puis à éliminer de manière appropriée le groupe protecteur.

22. Procédé selon la revendication 21, dans lequel la base nucléosidique de R₂ est une cytosine protégée par acétylation.

23. Procédé selon la revendication 21 ou 22, dans lequel la réaction est effectuée en présence d'une base.

24. Procédé selon la revendication 23, dans lequel la base est le 1-méthylimidazole.

25. Procédé de production d'un composé de formule (I), selon la revendication 1 ayant un groupe X qui représente -SO₂- dans lequel un composé de formule (IV), R₁SArOP(O)(OR₂)(OR₃), ou un composé de formule (V), R₁SOArOP(O)(OR₂)(OR₃), formules dans lesquelles R₁, Ar, R₂ et R₃ sont tels que définis à la revendication 1, est oxydé afin de convertir le groupe -S- ou -SO-, respectivement, en un groupe -SO₂-.

26. Procédé selon la revendication 25, dans lequel on utilise un peracide afin d'oxyder le composé de formule (IV) ou (V).

27. Composé de formule (IV), R₁SArOP(O)(OR₂)(OR₃), dans laquelle R₁, Ar, R₂ et R₃ sont tels que définis à la revendication 1.

28. Composé de formule (V), R₁SOArOP(O)(OR₂)(OR₃), dans laquelle R₁, Ar, R₂ et R₃ sont tels que définis à la revendication 1.

29. Composé de formule (I) selon l'une quelconque des revendications 1 à 20 pour utilisation dans une thérapie.

30. Utilisation d'un composé de formule (I) selon l'une quelconque des revendications 1 à 20 dans la préparation d'un médicament pour utilisation dans le traitement curatif ou préventif d'une infection par un rétrovirus humain.

31. Utilisation selon la revendication 30, dans laquelle le virus est le virus de l'immunodéficience humaine (VIH).

32. Méthode pour le traitement du sang afin d'inhiber ou d'empêcher la réplication virale dans celui-ci d'un rétrovirus humain qui comprend le traitement extra-corporel du sang avec un composé de formule (I) tel que défini à la revendication 1.

33. Méthode selon la revendication 32, dans laquelle le virus est le virus de l'immunodéficience humaine (VIH).

34. Composition pharmaceutique qui comprend un composé de formule (I) tel que défini dans l'une quelconque des revendications 1 à 20 avec un véhicule pharmaceutiquement acceptable pour celui-ci.

35. Composition pharmaceutique selon la revendication 34, sous la forme d'une dose.

## Revendications (Revendications pour l'(les) Etat(s) contractant(s) suivant(s): ES)

1. Procédé de production d'un composé de formule (I) 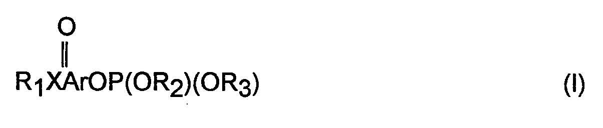 formule dans laquelle R₁ représente un groupe alkyle,
Ar représente un groupe phénylène substitué ou non substitué, X représente -SO₂- ou -CO- et R₂ et R₃ qui sont identiques représentent des parties de formule (a), (b), (c), (d), (e), (f), (g), (h) ou (i) : 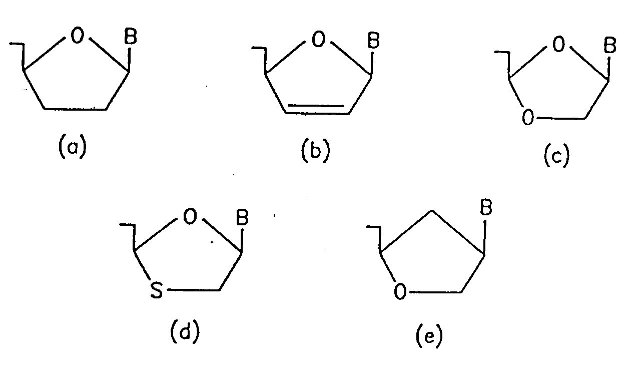 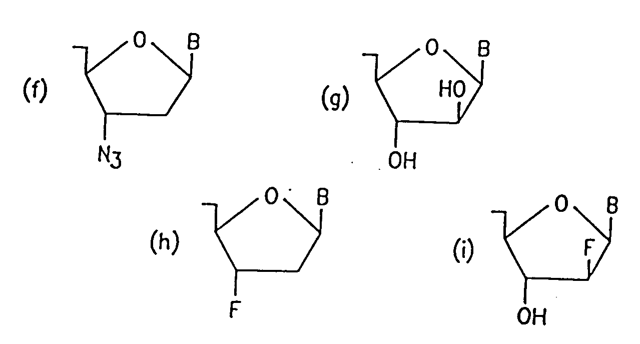 où B représente le résidu d'une base nucléosidique de formule (A), (G), (C), (H) ou (T) : 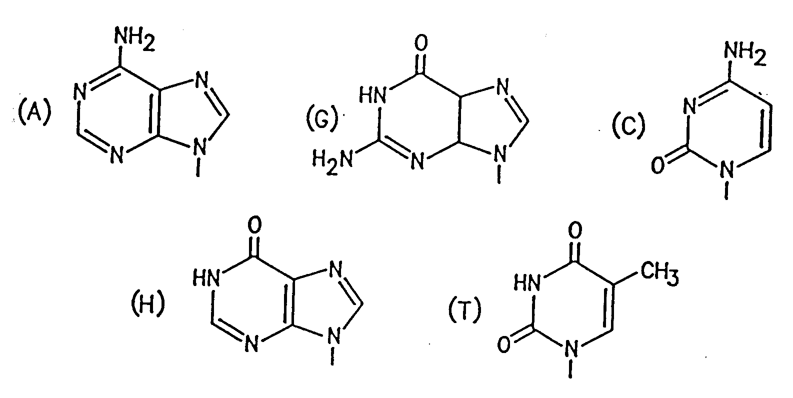 à condition que, lorsque R₂ et R₃ représentent tous les deux la partie de formule (a) alors B représente le résidu d'une base nucléosidique qui est de formule (A), (G), (C) ou (H), le composé étant éventuellement sous la forme d'un sel pharmaceutiquement acceptable, qui comprend une étape consistant à faire réagir un phosphonodihalogénure de formule (III), R₁XArOP(O)Y₂, dans laquelle R₁, X et Ar sont tels que définis pour le composé de formule (I) et Y représente un atome d'halogène, avec un composé de formule R₂OH dans laquelle R₂ correspond aux groupes identiques R₂ et R₃ dans le composé de formule (I) ou est un dérivé de celui-ci dans lequel un groupe de la base nucléosidique est protégée, puis à éliminer de manière appropriée le groupe protecteur.

2. Procédé selon la revendication 1, dans lequel la base nucléosidique dans R₂OH est protégée par acétylation lorsque R₂ est la cytosine.

3. Procédé selon la revendication 1 ou 2, dans lequel la réaction est réalisée en présence d'une base.

4. Procédé selon la revendication 3, dans lequel la base est le 1-méthylimidazole.

5. Procédé selon l'une quelconque des revendications 1 à 4, dans lequel X représente un groupe sulfonyle.

6. Procédé de production d'un composé de formule (I) 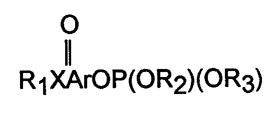 formule dans laquelle R₁ représente un groupe alkyle,
Ar représente un groupe phénylène substitué ou non substitué, X représente -SO₂- et R₂ et R₃ qui peuvent être identiques ou différents représentent des parties de formule (a), (b), (c), (d), (e), (f), (g), (h) ou (i) : 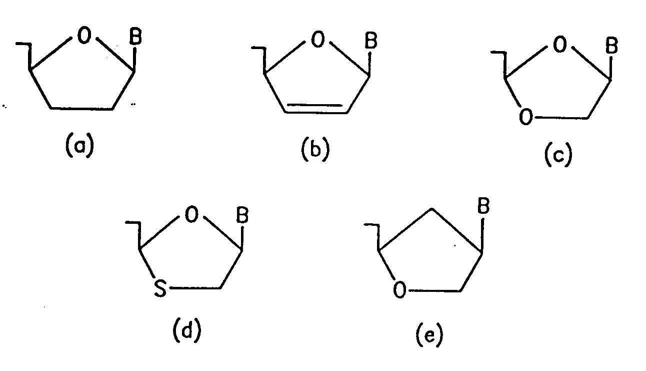 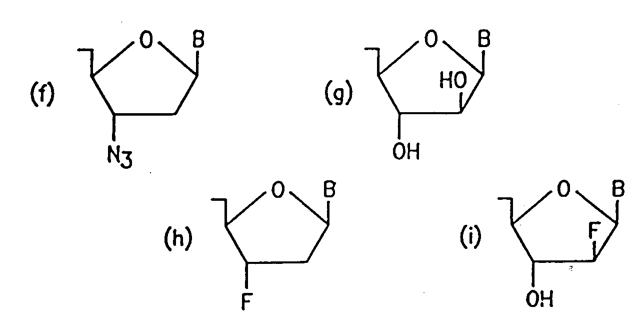 où B représente le résidu d'une base nucléosidique de formule (A), (G), (C), (H) ou (T) : 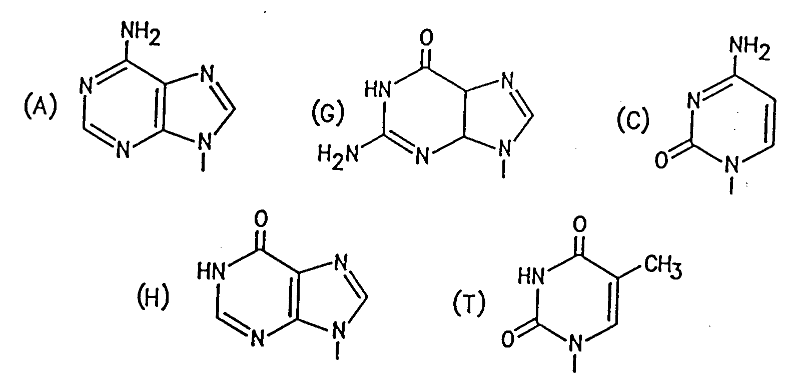 à condition que, lorsque R₂ et R₃ représentent tous les deux la partie de formule (a), alors B représente le résidu d'une base nucléosidique qui est de formule (A), (G), (C) ou (H), le composé étant éventuellement sous la forme d'un sel pharmaceutiquement acceptable, qui comprend une étape consistant à oxyder un composé de formule (IV), R₁SArOP(O)(OR₂)(OR₃), ou un composé de formule (V), R₁SOArOP(O)(OR₂)(OR₃), formules dans lesquelles R₁, Ar, R₂ et R₃ sont tels que définis pour le composé de formule (I), afin de convertir le groupe -S- ou -SO-, respectivement, en un groupe -SO₂-.

7. Procédé selon la revendication 6, dans lequel on utilise un peracide afin d'oxyder le composé de formule (IV) ou (V).

8. Procédé selon l'une quelconque des revendications 1 à 7, dans lequel Ar représente un groupe phénylène portant éventuellement un ou plusieurs substituants qui peuvent être identiques ou différents et qui sont un atome d'halogène, un groupe fluoroalkyle, alcoxy, fluoroalcoxy, carbalcoxy, amino ou amido.

9. Procédé selon la revendication 8, dans lequel le ou les différents substituants sont un atome d'halogène, un groupe alcoxy en C₁-₄, fluoroalcoxy en C₁₋₄, carboalcoxy en C₁₋₆, amino ou amido.

10. Procédé selon l'une quelconque des revendications 1 à 7, dans lequel Ar représente un groupe phénylène non substitué.

11. Procédé selon l'une quelconque des revendications précédentes, dans lequel le groupe R₁X est en para par rapport au groupe OP(O)(OR₂)(OR₃).

12. Procédé selon l'une quelconque des revendications précédentes, dans lequel R₁ représente un groupe alkyle en C₁₋₆.

13. Procédé selon la revendication 12, dans lequel R₁ représente un groupe méthyle.

14. Procédé selon l'une quelconque des revendications 1 à 6, dans lequel R₁XAr représente un groupe 4-(méthylsulfonyl)phényle.

15. Procédé selon l'un quelconque des revendications précédentes, dans lequel R₂ et R₃ l'un ou l'autre, ou indépendamment ou chacun, représentent des parties de formule (a), (b), (d) ou (f).

16. Procédé selon la revendication 15, dans lequel R₂ et R₃ représentent chacun la partie de formule (f).

17. Procédé selon l'une quelconque des revendications 1 à 14, dans lequel R₂ et R₃ l'un ou l'autre, ou indépendamment ou chacun, ont l'une quelconque des formules (i) à (x) :

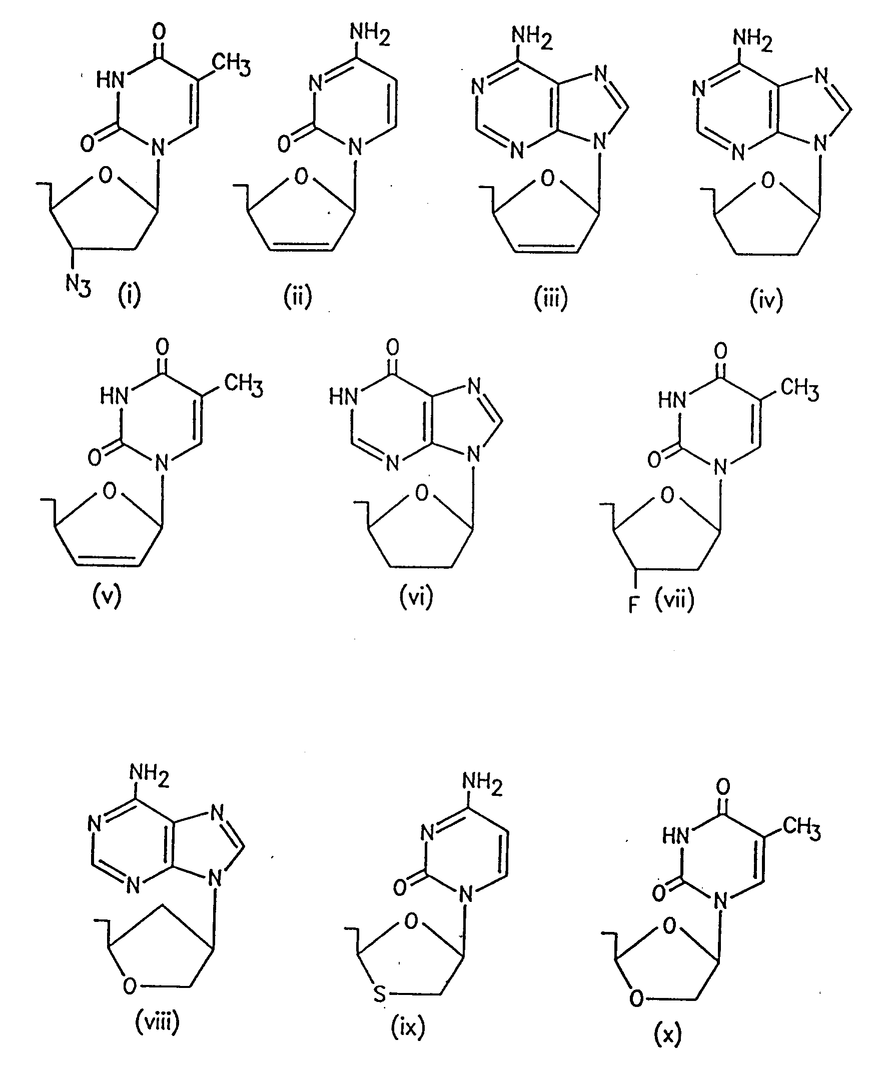

18. Procédé selon la revendication 17, dans lequel R₂ et R₃ l'un ou l'autre, ou indépendamment ou chacun, ont la formule (i), (v), (vi) ou (ix).

19. Procédé selon la revendication 18, dans lequel R₂ et R₃ ont chacun la formule (i).

20. Procédé selon la revendication 18, dans lequel R₂ et R₃ ont chacun la formule (v).

21. Procédé selon l'une quelconque des revendications 1 à 7, dans lequel le composé de formule (I) est le 4-(méthylsulfonyl)phényl bis(3'-azidothymidine-5'-yl)phosphate.

22. Procédé selon l'une quelconque des revendications 1 à 7, dans lequel le composé de formule (I) est le 4-(méthylsulfonyl)phényl bis(2',3'-didéshydro-2',3'-didésoxycytidine-5'-yl)phosphate.

23. Procédé selon l'une quelconque des revendications 1 à 7, dans lequel le composé de formule (I) est le 4-(méthylsulfonyl)phényl bis(2',3'-didéshydro-2',3'-didésoxyadénosine-5'-yl)phosphate.

24. Procédé selon l'une quelconque des revendications 1 à 7, dans lequel le composé de formule (I) est le 4-(méthylsulfonyl)phényl bis(2',3'-didésoxyadénosine-5'-yl)phosphate.

25. Procédé selon l'une quelconque des revendications 1 à 7, dans lequel le composé de formule (I) est le 4-(méthylsulfonyl)phényl bis(2',3'-didéshydro-3'-désoxythymidine-5'-yl)phosphate.
